(19) Europäisches Patentamt
European Patent Office
Office européen des brevets

(11) Veröffentlichungsnummer : **0 359 710 B1**

(12) # EUROPÄISCHE PATENTSCHRIFT

(45) Veröffentlichungstag der Patentschrift : **16.06.93 Patentblatt 93/24**

(21) Anmeldenummer : **89810667.9**

(22) Anmeldetag : **07.09.89**

(51) Int. Cl.$^5$ : **C07D 413/12**, C08K 5/45, C09D 11/02, G03C 1/40, B41M 5/132

(54) **Optische Aufheller.**

Die Akte enthält technische Angaben, die nach dem Eingang der Anmeldung eingereicht wurden und die nicht in dieser Patentschrift enthalten sind.

(30) Priorität : **16.09.88 CH 3456/88**

(43) Veröffentlichungstag der Anmeldung : **21.03.90 Patentblatt 90/12**

(45) Bekanntmachung des Hinweises auf die Patenterteilung : **16.06.93 Patentblatt 93/24**

(84) Benannte Vertragsstaaten : **BE DE FR GB IT NL**

(56) Entgegenhaltungen :
**EP-A- 0 099 861**
**CH-A- 492 818**
**GB-A- 1 159 180**
**US-A- 3 136 773**
**US-A- 3 449 257**

(56) Entgegenhaltungen :
**US-A- 4 267 343**
**CHEMICAL ABSTRACTS, Band 110, Nr. 4, 23. Januar 1989 Columbus, Ohio, USA IWAGAKI, MASARU et al.: "Image receptor unit for thermally developed silver halide color photographic materials with increased background brightness." Seite 500, Spalte 1, Zusammenfassung-Nr. 31 333e**
**CHEMICAL ABSTRACTS, Band 76, Nr. 24, 12. Juni 1972, Columbus, Ohio, USA NOGUCHI, TAMEHIKO et al.: "Fluorescent whitening agents for polyester and polyamide fibers." Seite 84, Spalte 2, Zusammenfassung-Nr. 142 407f**

(73) Patentinhaber : **CIBA-GEIGY AG**
**Klybeckstrasse 141**
**CH-4002 Basel (CH)**

(72) Erfinder : **Leppard, David G., Dr.**
**Route de Bourguillon 6**
**CH-1723 Marly (CH)**
Erfinder : **Vieira, Eric, Dr.**
**Rue d'Or 5**
**CH-1700 Fribourg (CH)**

**EP 0 359 710 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue 2,5-Bis-benzoxazolyl-thiophene, die als optische Aufheller für organische Materialien und insbesondere für Abbildungs- und Aufzeichnungsmaterialien eingesetzt werden können.

2,5-Bis-benzoxazolyl-thiophene sind als optische Aufheller für organische Materialien seit langem bekannt und einige Vertreter dieser Klasse sind im Handel erhältlich. 2,5-Bis-benzoxazolyl-thiophene, deren Herstellung und Verwendung als optische Aufheller sind beispielsweise in den US-A 3,136,773, 3,449,257 und 4,267,343 beschrieben. Ebenso ist es bekannt, optische Aufheller, auch solche der genannten Klasse, photographischen Aufzeichnungsmaterialien, z.B. Photopapieren, zuzusetzen, um deren Weisseindruck bzw. Brillanz zu verbessern. Siehe z.B. US-A 3,135,762, 3,416,923, 3,418,127 und 3,449,257, GB-A 1 072 915 und JP-A 61-124939. Ein besonderes Problem stellt dabei die Einarbeitung der Aufheller in die entsprechende Schicht des Aufzeichnungsmaterials dar. Es besteht daher ein Bedarf an Verbindungen, die als optische Aufheller leicht in photographische Aufzeichnungsmaterialien einzuarbeiten sind und die in solchen Materialien gute Aufhellerwirkung entfalten. Weiterhin besteht nach wie vor ganz allgemein auch ein Bedarf an neuen optischen Aufhellern für verschiedenste Anwendungen.

Ein erster Gegenstand der vorliegenden Erfindung sind daher neue Verbindungen der Formel

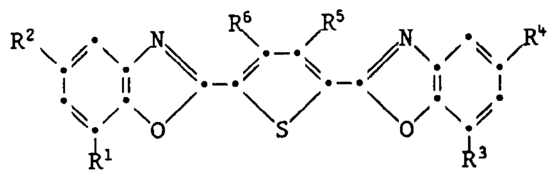

(I) ,

worin

$R^1$ und $R^3$ unabhängig voneinander $C_1$-$C_8$-Alkyl, Cyclopentyl, Cyclohexyl, $C_7$-$C_9$-Aralkyl, Phenyl oder eine Gruppe der Formel

$$-C_mH_{2m+1-q}(X)_q \qquad (II)$$

bedeuten,

worin m für eine Zahl von 0-10 und q für 1 oder 2 stehen, wobei im Fall m = 0 q = 1 ist, $R^2$ und $R^4$ unabhängig voneinander jeweils eine Gruppe der Formel II bedeuten,

oder $R^2$ und $R^4$ für den Fall, dass $R^1$ und $R^3$ jeweils eine Gruppe der Formel II darstellen, unabhängig voneinander auch $C_1$-$C_8$-Alkyl, Cyclopentyl, Cyclohexyl, $C_7$-$C_9$-Aralkyl oder Phenyl bedeuten können,

$R^5$ und $R^6$ unabhängig voneinander Wasserstoff, Methyl, Ethyl oder zusammen Trimethylen oder Tetramethylen bedeuten,

und X

a) eine Gruppe der Formel $-COOR^9$, worin $R^9$ Wasserstoff, ein Alkalimetallion oder das Aequivalent eines Erdalkalimetallions, $C_1$-$C_{30}$-Alkyl, $C_1$-$C_{18}$-Hydroxyalkyl, durch mindestens ein -O- unterbrochenes $C_3$-$C_{20}$-Alkyl oder -Hydroxyalkyl, unsubstituiertes oder durch OH oder/und $C_1$-$C_4$-Alkyl substituiertes $C_5$-$C_{12}$-Cycloalkyl, $C_2$-$C_{30}$-Alkenyl, $C_7$-$C_{15}$-Aralkyl, Glycidyl, Furfuryl oder eine Gruppe $-CH_2-CH(OH)-R^{10}$ darstellt, worin $R^{10}$ für Wasserstoff, $C_1$-$C_{12}$-Alkyl, $C_7$-$C_9$-Aralkyl oder $-CH_2OR^{11}$ steht, wobei $R^{11}$ $C_1$-$C_{18}$-Alkyl, Cyclohexyl, Phenyl, Tolyl oder Benzyl ist, oder $R^9$ eine Gruppe der Formel $-(CH_2)_pP(O)(OR^{16})(OR^{17})$ oder $-(CH_2)_pP(O)(OR^{16})-R^{18}$, worin p 1 bis 6 ist und $R^{16}$, $R^{17}$ und $R^{18}$ wie nachstehend unter d) definiert sind, darstellt;

b) eine Gruppe der Formel $-CONR^{12}R^{13}$,

worin $R^{12}$ und $R^{13}$ unabhängig voneinander Wasserstoff, $C_1$-$C_{18}$-Alkyl, durch mindestens ein -O- unterbrochenes $C_3$-$C_{20}$-Alkyl, unsubstituiertes oder durch $C_1$-$C_4$-Alkyl oder/und OH substituiertes $C_5$-$C_{12}$-Cycloalkyl, Phenyl oder durch $C_1$-$C_{12}$-Alkyl, $C_1$-$C_4$-Alkoxy oder/und Halogen substituiertes Phenyl, $C_3$-$C_{18}$-Alkenyl, $C_7$-$C_{12}$-Aralkyl oder $C_2$-$C_4$-Hydroxyalkyl sind, oder $R^{12}$ und $R^{13}$ zusammen $C_4$-$C_5$-Alkylen oder durch -O- oder $-N(R^{15})$ unterbrochenes $C_4$-$C_6$-Alkylen darstellen, wobei $R^{15}$ Wasserstoff, $C_1$-$C_{18}$-Alkyl, Cyclohexyl, $C_2$-$C_6$-Alkenyl, Phenyl oder $C_7$-$C_{12}$-Alkylphenyl oder $C_7$-$C_{12}$-Aralkyl bedeutet;

c) eine Gruppe der Formel

$$-O\overset{O}{\overset{\|}{C}}-R^{20},$$

worin $R^{20}$ $C_1$-$C_{18}$-Alkyl, Cyclohexyl, $C_2$-$C_{18}$-Alkenyl, Phenyl oder $C_7$-$C_{12}$-Alkylphenyl oder $C_7$-$C_{12}$-Aralkyl bedeutet;

d) eine Gruppe der Formel $-P(O)(OR^{16})(OR^{17})$ oder $-P(O)(OR^{16})-R^{18}$, worin $R^{16}$ Wasserstoff oder $C_1$-$C_{12}$-Alkyl, $R^{17}$ $C_1$-$C_{12}$-Alkyl und $R^{18}$ $C_1$-$C_{12}$-Alkyl oder Phenyl darstellen; oder

e) eine Gruppe der Formel

$$-\overset{O}{\overset{\|}{C}}-R^{19},$$

worin $R^{19}$ $C_1$-$C_{12}$-Alkyl darstellt, bedeutet.

Unter den Gruppen der Formel II sind z.B. solche erwähnenswert, die der Formel

$$-\underset{R^8}{\overset{R^7}{\overset{|}{\underset{|}{C}}}}-C_nH_{2n+1-k}(X)_k \qquad \text{(III)}$$

entsprechen, worin n für eine Zahl von 2-8 und k für 1 oder 2 stehen, $R^7$ und $R^8$ unabhängig voneinander $C_1$-$C_5$-Alkyl sind oder $R^7$ zusammen mit dem Rest $C_nH_{2n+1-k}$ einen $C_5$-$C_6$-Cycloalkylring bildet. Wenn $R^1$ und $R^3$ eine Gruppe der Formel II bedeuten, handelt es sich dabei bevorzugt um eine Gruppe der Formel III.

Soweit die Substituenten in Formel I Alkyl bedeuten, handelt es sich dabei um geradkettiges oder verzweigtes Alkyl. Das gleiche gilt für substituierte oder durch Heteroatome unterbrochene Alkylgruppen. Ebenso können Alkylengruppen sowie substituierte oder durch Heteroatome unterbrochene Alkylengruppen geradkettig oder verzweigt sein.

Sofern R-Substituenten $C_1$-$C_{30}$-Alkyl bedeuten, handelt es sich dabei beispielsweise um Methyl, Ethyl, n-Propyl, Isopropyl, n-Butyl, sek-Butyl, t-Butyl, n-Pentyl, t-Amyl, n-Hexyl, n-Heptyl, n-Octyl, 2-Ethylhexyl, 1,1,3,3-Tetramethylbutyl, n-Nonyl, n-Decyl, 2,7-Dimethyloctyl, n-Dodecyl, n-Tridecyl, n-Tetradecyl, n-Hexadecyl, n-Octadecyl, Eicosyl, Docosyl oder Triacontyl. Beispiele für Alkyl-R-Substituenten mit kürzerer Kette sind die vorstehend genannten bis zur entsprechenden Anzahl der C-Atome. $R^1$ bzw. $R^3$ sind vorzugsweise $C_1$-$C_4$-Alkyl, insbesondere t-Butyl. $R^7$ und $R^8$ sind vorzugsweise $C_1$-$C_2$-Alkyl, insbesondere Methyl. $R^9$ als Alkyl hat vorzugsweise 1-18 C-Atome.

$R^1$, $R^2$, $R^3$, $R^4$ und $R^{10}$ als $C_7$-$C_9$-Aralkyl sind z.B. Benzyl, Phenethyl, α-Methylbenzyl und α,α-Dimethylbenzyl.

In Formel III ist n beispielsweise 2-4, insbesondere 3 und k vorzugsweise 1. In Formel II ist m insbesondere 0-6 und q insbesondere 1. Wenn k bzw. q 1 ist, ist X vorzugsweise endständig. Falls q oder k 2 ist, können die X-Gruppen an das gleiche oder an verschiedene C-Atome gebunden sein.

Als durch ein oder mehrere -O- unterbrochene $C_3$-$C_{20}$-Alkylgruppen $R^9$, $R^{12}$ und $R^{13}$ seien beispielsweise 2-Methoxyethyl, 2-Ethoxyethyl, 2-n-Propoxy ethyl, 2-n-Butoxyethyl, oder

$$-(CH_2\underset{R_o}{\underset{|}{C}}H-O)_x-R_o'$$

genannt, worin $R_o$ H oder $CH_3$, $R_o'$ $C_1$-$C_4$-Alkyl und x 1 bis 6 bedeuten, insbesondere 2-(2-Methoxyethyl)ethyl, 2-(2-Ethoxyethyl)ethyl und 2-(2-Butoxyethyl)ethyl. Als Beispiele für gegebenenfalls durch -O- unterbrochenes Hydroxyalkyl $R^9$ können die für "Alkyl" und "durch -O- unterbrochenes Alkyl" angeführten Reste dienen, die durch 1 oder mehrere, vorzugsweise 1 bis 3, insbesondere 1 OH-Gruppe substituiert sind.

Gegebenenfalls OH und/oder $C_1$-$C_4$-Alkyl-substituiertes $C_5$-$C_{12}$-Cycloalkyl $R^{19}$, $R^{12}$ und $R^{13}$ kann beispielsweise Cyclopentyl, Cyclohexyl, Cycloheptyl, Cyclooctyl, Adamantyl, Cyclododecyl, 2- oder 4-Hydroxycyclohexyl oder 2-, 3- oder 4-Methylcyclohexyl bedeuten. Bevorzugt sind die unsubstituierten Cycloalkylgruppen, insbesondere Cyclopentyl und vor allem Cyclohexyl, sowie 2- oder 4-Methylcyclohexyl.

$R^9$ als $C_2$-$C_{30}$-Alkenyl ist beispielsweise Vinyl, Allyl, n-But-2-enyl, 2-Methylprop-2-enyl, n-Pent-2-enyl, n-Hex-2-enyl, n-Hexa-2,4-dienyl, n-Dec-9-enyl, n-Undecen-10-yl, n-Octadec-9-enyl oder n-Octadec-17-enyl.

Für andere R-Reste, die Alkenyl bedeuten, gelten die gleichen Reste als Beispiele, jeweils entsprechend ihrer möglichen Länge der C-Kette. $R^9$ als Alkenyl hat vorzugsweise 1-18 C-Atome.

$R^9$, $R^{12}$, $R^{13}$ und $R^{15}$ als $C_7$-$C_{15}$-Aralkyl bzw. $C_7$-$C_{12}$-Aralkyl bedeuten z.B. Naphthyl-$C_1$-$C_5$-alkyl oder Phenyl-$C_1$-$C_9$-alkyl bzw. Naphthyl-$C_1$-$C_2$-alkyl oder Phenyl-$C_1$-$C_6$-alkyl, vorzugsweise Phenyl-$C_1$-$C_3$-alkyl. Beispiele für letzteres sind weiter oben unter $C_7$-$C_9$-Aralkyl angegeben.

Halogen bedeutet Cl, F oder Br, insbesonders Cl.

Sofern $R^{12}$ und $R^{13}$ $C_4$-$C_5$-Alkylen bedeuten, bilden sie mit dem N-Atom einen Pyrrolidin- oder Piperidinring, in der Bedeutung von durch -O- oder -N($R^{15}$)- unterbrochenem $C_4$-$C_6$-Alkylen bilden sie mit dem N-Atom einen 5-7-gliedrigen Heterocyclus, der z.B. 2 N- oder ein N- und ein O-Atom als Ringglieder enthält, beispielsweise einen Piperazin- oder Morpholinring, wobei der Piperazinring am N-Atom einen Substituenten $R^{15}$ tragen kann.

In Formel I sind vorzugsweise $R^1$ und $R^3$ sowie $R^2$ und $R^4$ jeweils gleich.

In bevorzugten Verbindungen der Formel I ist $R^1$ bzw. $R^3$ $C_1$-$C_4$-Alkyl, Cyclohexyl oder eine Gruppe der Formel II (vor allem eine Gruppe der Formel III), insbesondere $C_1$-$C_4$-Alkyl (besonders bevorzugt t-Butyl), Cyclohexyl oder eine Gruppe der Formel

$$-\underset{CH_3}{\overset{CH_3}{\underset{|}{\overset{|}{C}}}}-(CH_2)_3COOR^9 .$$

$R^1$ und $R^3$ sind dabei ganz besonders bevorzugt $C_1$-$C_4$-Alkyl, insbesondere t-Butyl.

Hervorzuheben sind ferner Verbindungen der Formel I, worin $R^1$ und $R^3$ $C_1$-$C_4$-Alkyl, Cyclohexyl, Benzyl oder eine Gruppe der Formel II, insbesondere eine Gruppe der Formel III, $R^2$ und $R^4$ eine Gruppe der Formel II, oder, wenn $R^1$ für eine Gruppe der Formel II steht, auch $C_1$-$C_8$-Alkyl, Cyclopentyl, Cyclohexyl oder Phenyl-$C_1$-$C_3$-alkyl, m 0 bis 6, $R^5$ und $R^6$ unabhängig voneinander Wasserstoff, Methyl oder Ethyl, $R^7$ und $R^8$ $C_1$-$C_4$-Alkyl, insbesondere jeweils Methyl, $R^9$ Wasserstoff, $C_1$-$C_{18}$-Alkyl, Cyclopentyl, Cyclohexyl, $C_2$-$C_{18}$-Alkenyl, Phenyl-$C_1$-$C_3$-alkyl, Furfuryl, durch 1 bis 4 -O- unterbrochenes $C_3$-$C_{18}$-Alkyl oder $-(CH_2)_pP(O)(OC_1\text{-}C_6\text{-Alkyl})_2$, worin p 1 bis 6 ist, $R^{12}$ und $R^{13}$ unabhängig voneinander Wasserstoff, $C_1$-$C_{18}$-Alkyl, Cyclopentyl, Cyclohexyl, Phenyl, durch $C_1$-$C_4$-Alkyl, Methoxy oder/und Chlor substituiertes Phenyl, $C_3$-$C_{18}$-Alkenyl, Benzyl, $C_2$-$C_4$-Hydroxyalkyl, oder $R^{12}$ und $R^{13}$ zusammen $C_4$-$C_5$-Alkylen oder durch -O-, -NH- oder -N($CH_3$)- unterbrochenes $C_4$-$C_5$-Alkylen, $R^{15}$ $C_1$-$C_8$-Alkyl, Cyclohexyl, $C_2$-$C_6$-Alkenyl, Phenyl, Tolyl oder Benzyl, $R^{16}$ Wasserstoff oder $C_1$-$C_8$-Alkyl, $R^{17}$ $C_1$-$C_8$-Alkyl, $R^{18}$ $C_1$-$C_8$-Alkyl und $R^{19}$ $C_1$-$C_8$-Alkyl bedeuten.

Der Substituent X in Formel I ist insbesondere eine Gruppe -CONR$^{12}$R$^{13}$ oder -COOR$^9$, wobei letztere bevorzugt ist.

Besonders zu erwähnen sind auch Verbindungen der Formel I, worin $R^2$ bzw. $R^4$ eine Gruppe der Formel II ist, worin q 1 oder 2 und m für 0 bis 6 stehen, oder $R^2$ bzw. $R^4$ $C_1$-$C_8$-Alkyl, Cyclopentyl, Cyclohexyl oder Phenyl-$C_1$-$C_3$-alkyl bedeutet, wenn $R^1$ bzw. $R^3$ eine Gruppe der Formel II bzw. der Formel III ist. Auch in diesem Fall sind $R^2$ und $R^4$ vorzugsweise gleich.

Die Substituenten $R^5$ und $R^6$ bedeuten unabhängig voneinander vorzugsweise Wasserstoff, Methyl oder Ethyl.

Von besonderer praktischer Bedeutung sind die Verbindungen der Formel I, worin $R^1$ und $R^3$ gleich sind und $C_1$-$C_4$-Alkyl oder eine Gruppe der Formel II (und vorzugsweise eine Gruppe der Formel III mit $R^7$ und $R^8$ = $C_1$-$C_4$-Alkyl, n = 2 bis 4 und k = 1) bedeuten, $R^2$ und $R^4$ gleich sind und für eine Gruppe der Formel II oder, wenn $R^1$ und $R^3$ Gruppen der Formel II bzw. III sind, auch für $C_1$-$C_4$-Alkyl stehen, m 0-6 und X eine Gruppe der Formel -COOR$^9$ oder -CONR$^{12}$R$^{13}$ bedeuten, worin $R^9$ Wasserstoff, $C_1$-$C_{18}$-Alkyl, $C_3$-$C_{18}$-Alkenyl, Furfuryl, Cyclohexyl, Phenyl-$C_1$-$C_3$-alkyl, durch 1-4 -O- unterbrochenes $C_3$-$C_{18}$-Alkyl oder $-(CH_2)_{p'}P(O)(OC_1\text{-}C_6\text{-Alkyl})_2$ ist, worin p′ 1-4 bedeutet und $R^{12}$ und $R^{13}$ unabhängig voneinander Wasserstoff oder $C_1$-$C_{12}$-Alkyl sind; wobei jene Verbindungen hervorzuheben sind, worin $R^1$ und $R^3$ $C_1$-$C_4$-Alkyl oder eine Gruppe der Formel II (und vorzugsweise der Formel III, worin $R^7$ und $R^8$ Methyl oder Ethyl, n 2 und k 1 sind) bedeuten, $R^2$ und $R^4$ für Gruppen der Formel II stehen oder, wenn $R^1$ und $R^3$ Gruppen der Formel II bzw. III sind, auch für $C_1$-$C_4$-Alkyl stehen, m 0 bis 6 und X eine Gruppe -COOR$^9$ bedeuten und $R^9$ $C_1$-$C_{18}$-Alkyl, durch 1-3 -O- unterbrochenes $C_3$-$C_{12}$-Alkyl oder $C_3$-$C_{18}$-Alkenyl bedeutet.

Wie bereits erwähnt, sind jene Verbindungen der Formel I besonders vorteilhaft, in denen $R^1$ und $R^3$ jeweils t-Butyl bedeuten.

Die Thiophenverbindungen der Formel I zeigen in gelöstem oder fein verteiltem Zustand eine ausgeprägte Fluoreszenz. Sie können daher zum optischen Aufhellen einer Reihe von Materialien, insbesondere von organischen Materialien, verwendet werden. Weitere Gegenstände der vorliegenden Erfindung sind daher die Verwendung der Verbindungen der Formel I zum optischen Aufhellen von organischen Materialien, ferner organische Materialien, enthaltend mindestens eine Verbindung der Formel I sowie ein Verfahren zum optischen Aufhellen von organischen Materialien, das dadurch gekennzeichnet ist, dass man diesen Materialien mindestens eine Verbindung der Formel I einverleibt oder auf diese aufbringt.

Zu den organischen Materialien, die mit Hilfe von Verbindungen der Formel I erfindungsgemäss optisch aufgehellt werden können, gehören synthetische, halbsynthetische oder natürliche, insbesondere polymere, Materialien. Hierfür seien beispielsweise, ohne dass durch die nachfolgende Uebersicht irgendeine Beschränkung hierauf ausgedrückt werden soll,die folgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte, wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von $\alpha$,ß-ungesättigten Carbonsäuren oder Derivaten solcher Carbonsäuren, insbesondere von Acrylverbindungen (wie z.B. Acrylestern, Acrylsäuren, Acrylnitril, Acrylamiden und deren Derivaten oder deren Methacryl-Analoga), von Olefin-Kohlenwasserstoffen (wie z.B. Ethylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),

b) Polymerisationsprodukte, die durch Ringöffnung erhältlich sind, z.B. Polyamide vom Polycaprolactam-Typ, ferner Polymere, die sowohl über Polyaddition als auch Polykondensation erhältich sind, wie Polyether oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Ethylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiamin-adipat), Maleinatharze, Melaminharze, deren Vorkondensate und Analoga, Polycarbonate, Silikone,

d) Polyadditionsprodukte wie Polyurethane (vernetzt und unvernetzt), Epoxidharze.

II. Halbsynthetische organische Materialien, z.B. Celluloseester verschiedener Veresterungsgrade (sogenanntes 2 1/2-Acetat, Triacetat) oder Celluloseether, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen, wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d.h. beispielsweise als vorwiegend dreidimensional ausgedehnte Körper, wie Platten, Profile, Spritzgussformlinge, verschiedenartige Werkstücke, Schnitzel, Granulate oder Schaumstoffe, ferner als vorwiegend zweidimensional ausgebildete Körper, wie Filme, Folien, Lacke, Ueberzüge, Imprägnierungen und Beschichtungen oder als vorwiegend eindimensional ausgebildete Körper, wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäss anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wässerigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. die Beahndlung wird üblicherweise bei Temperaturen

von etwa 20 bis 140°C, beispielsweise bei Siedetemperatur des Bades oder in deren Nähe (etwa 90°C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen erfindungsgemässen optischen Aufheller können beispielsweise auch zur Aufhellung von Papiermassen, unter anderem auch in Gegenwart von z.B. kationischen Retentionsmitteln und anderen Zusatzstoffen eingesetzt werden.

Die neuen optischen Aufhellmittel gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

- Zugabe zu den Ausgangssubstanzen (z.B. Monomeren) oder Zwischenprodukten (z.B. Vorkondensaten, Praepolymeren), d.h. vor oder während der Polymerisation, Polykondensation oder Polyaddition,
- Aufpudern auf Polymerisatschnitzel oder Granulate für Spinnmassen,
- Badfärbung von Polymerisatschnitzeln oder Granulaten für Spinnmassen,
- dosierte Zugabe zu Spinnschmelzen oder Spinnlösungen,
- Applikation auf Spinnkabel vor dem Verstrecken.

Die neuen optischen Aufhellmittel gemäss vorliegender Erfindung können beispielsweise auch in folgenden Anwendungsformen eingesetzt werden:

a) Mischungen mit Farbstoffen (Nuancierung) oder Pigmenten (Farb- oder insbesondere z.B. Weisspigmenten) oder als Zusatz zu Färbebädern, Druck-, Aetz- oder Reservepasten. Ferner auch zur Nachbehandlung von Färbungen, Drucken oder Aetzdrucken,

b) in Mischungen mit sogenannten "Carriern", Netzmitteln, Weichmachern, Quellmitteln, Antioxidantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie "wash- and -wear", "permanent-press" "no-iron") ferner Flammfest-, Weichgriff-, Schmutzablöse ("anti-soiling")oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu den verschiedensten industriellen Produkten, um dieselben marktfähiger zu machen (z.B. Aspektverbesserung von Seifen, Waschmitteln, Pigmenten),

f) in Kombination mit anderen, optisch aufhellend wirkenden Substanzen,

g) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser, z.B. als Nachbehandlung von nassversponnenen Polyacrylfasern im sogenannten Gelzustand,

h) als Scintillatoren, für verschiedene Zwecke photographischer Art, wie z.B. für elektrophotographische Reproduktion oder Supersensiblisierung,

i) je nach Substitution als Laser-Farbstoffe,

j) in Verstärkerfolien ("Intensifying screens") für die medizinische Radiologie; sie eignen sich jedoch besonders für die Verwendung in Strahlungsspeicherfolien ("Radiation image storage screens"), die mit Licht angeregt werden können, wie sie z.B. in Research Disclosure 28704 (März 1988) beschrieben sind,

k) als Farbstoffzusatz in Kühlmitteln und als Leckdetektionsmittel für Kühlsysteme.

Wird das Aufhellverfahren mit Textil-Behandlungs- oder -Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemässen Aufhellern zweckmässig in der Weise, dass man diese Fasern mit den wässerigen Dispersionen (gegebenenfalls auch Lösungen) der

Aufhellmittel bei Temperaturen unter 75°C, z.B. bei Raumtemperatur, imprägniert und einer trockenen Wärmebehandlung bei Temperaturen über 100°C unterwirft, wobei es sich im allgemeinen empfiehlt, das Fasermaterial vorher noch bei mässig erhöhter Temperatur, z.B. bei mindestens 60°C bis etwa 130°C, zu trocknen. Die Wärmebehandlung in trockenem Zustande erfolgt dann vorteilhaft bei Temperaturen zwischen 120 und 225°C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,001 und 0,5, insbesondere 0,01 bis 0,5, z.B. 0,05 bis 0,2 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphaten oder Alkalimetallsilicaten.

Ferner können die erfindungsgemässen Verbindungen der Formel I auch in Ueberzugsmaterialien, insbesondere in Lacken, eingesetzt werden. Ein praktisch wichtiges Anwendungsgebiet sind Dispersionsfarben. Gegenstand der Erfindung sind daher auch die genannten Materialien, die mindestens eine Verbindung der Formel I enthalten. Besonders vorteilhaft handelt es sich bei den Ueberzugsmaterialien und Lacken um hell-, insbesondere weiss-pigmentierte Systeme, wo der Aufhelleffekt gut zur Geltung kommt.

Besonders bevorzugt ist die Anwendung der erfindungsgemässen Verbindungen der Formel I in Lacken aller Art, die z.B. auf Metall-, Kunststoff- oder Holzteilen appliziert werden.

Beispiele dafür sind:

- Lacke auf Basis von heissvernetzbaren Polyester-, Alkyd-, Acryl-, Acryl/Alkyd- oder Melaminharzen, die bei Temperaturen von mehr als 80°C eingebrannt werden, gegebenenfalls durch zusätzliche Anwendung saurer Katalysatoren
- 2-Komponenten-Polyurethanlacke auf Basis von hydroxylgruppenhaltigem Acrylatharz und aliphatischen oder aromatischen Isocyanaten oder auch auf Basis von hydroxylgruppenhaltigen Polyesterharzen und/oder Polyetherharzen und aliphatischen oder aromatischen Isocyanaten
- 1-Komponenten-Polyurethanlacke auf Basis von blockierten Isocyanaten, die während des Einbrennvorgangs deblockiert werden
- 2-Komponenten-Lacke auf Basis von (Poly)ketiminen und aliphatischen oder aromatischen Isocyanaten
- 2-Komponenten-Isocyanatfreie-Lacke auf Basis von carboxylgruppenhaltigen oder amingruppenhaltigen Polyacrylaten und Epoxiden
- 2-Komponenten-Lacke, bei denen eine Komponente aus Acrylatharzen besteht, die mit Anhydridgruppen modifiziert sind
- 2-Komponenten-Lacke auf Basis von (Poly)ketiminen und ungesättigten Polyacrylaten oder Polyacetoacetatharzen
- 2-Komponenten-Lacke auf Basis von (Poly)ketiminen und Methyl-Acrylamido-Glykolat-Methylester
- 2-Komponenten-Lacke auf Basis von Oxazolidinen und ungesättigten Polyacrylaten oder aliphatischen oder aromatischen Isocyanaten
- 2-Komponenten-Lacke auf Basis von ungesättigten Polyacrylaten und Malonaten
- thermoplastische Polyacrylatsysteme auf Basis von thermoplastischen Polyacrylatharzen oder fremdvernetzenden Polyacrylatharzen in Kombination mit veretherten Melaminharzen. Fremdvernetzende Polyacrylatharze sind z.B. beschrieben in H. Kittel, Lehrbuch der Lacke und Beschichtungen, Bd I, Teil 2, Seite 712
- Lacksysteme auf Basis siloxanmodifizierter Acrylate
- bei Raumtemperatur härtbare Systeme auf Basis von Harzen aus der Gruppe unmodifizierter oder modifizierter Alkydharze, thermoplastischer Acrylharze, Acryl-Alkydharze, Polyesterharze und vernetzter Epoxidharze sowie epoxyvernetzter Acryl- und Polyesterharze
- Beschichtungen auf Basis von Polyvinylidenfluoriden.

Die Lacke liegen in der Praxis entweder in organischer oder wässriger Phase vor. Ebenso können aber auch die erfindungsgemässen Verbindungen in an sich bekannten Pulverlacken eingesetzt werden.

Des weiteren ist die Anwendung der erfindungsgemässen Verbindungen in Lacken bevorzugt, bei denen

die Einleitung des Vernetzungsvorgangs durch energiereiche Strahlung verursacht wird.

Beispiele dafür sind Komponenten, die aus einer oder mehreren ungesättigten Verbindungen bestehen. Diese Verbindungen können eine oder mehrere ungesättigte olefinische Doppelbindungen enthalten und in niedermolekularer Form (monomer) oder höhermolekularer Form (oligomer) vorliegen.

Die Lacksysteme sind dem Fachmann bekannt und können als Ein-, Zwei- oder Dreikomponentensysteme vorliegen.

Die Härtung erfolgt durch die dem Fachmann bekannten Methoden, z.B. durch Zugabe eines Photoinitiators und dessen Aktivierung in einem bestimmten Wellenlängenbereich oder durch Elektronenstrahlhärtung.

Sämtliche Lacke können dabei als 1-Schicht oder 2-Schichtlacke appliziert werden, wobei unter 2-Schichtlacken solche verstanden werden, die durch das Auftragen eines Klarlackes auf einem Metalleffekt- oder unipigmentierten Basislack erhalten werden.

Die erfindungsgemässen Verbindungen sind dabei im pigmentierten (metallic- oder unipigmentiert) Basislack und/oder Klarlack enthalten, vorzugsweise im Klarlack.

Die erfindungsgemässen Verbindungen werden zweckmässig in die Nass-, Pulver- oder strahlungshärtbaren Lacke vor dem Einbrennen bzw. Aushärten eingearbeitet.

Das Aufbringen der Lacke auf ein Substrat kann mit Hilfe aller herkömmlichen Verfahren geschehen, die dem Fachmann bekannt sind, z.B. durch Streichen, Besprühen, Giessen, Tauchen oder elektrophoretisches Aufbringen.

Weitere Substrate, die erfindungsgemässe Verbindungen enthalten können, sind Druckfarben und Drucktinten, die die üblichen Pigmente anorganischer oder organischer Provenienz enthalten können.

Obwohl die erfindungsgemässen Verbindungen in den vorstehend genannten Substraten und Applikationsverfahren gute Aufhelleffekte bewirken, liegt die am meisten bevorzugte Anwendung im Bereich von Abbildungs- und Aufzeichnungsmaterialien, insbesondere im Bereich von photographischen Aufzeichnungsmaterialien. Ein besonders bevorzugter Gegenstand der vorliegenden Erfindung sind daher auch Abbildungs- und Aufzeichungsmaterialien, insbesondere photographische Aufzeichnungsmaterialien, die mindestens eine Verbindung der Formel I enthalten. Als Aufzeichnungsmaterialien sind insbesondere Photopapiere zu erwähnen, wobei es sich um solche für schwarz-weisse oder farbige Kopien handeln kann.

Im Fall von Photopapieren können die Verbindungen der Formel I in die Papierbasis selbst oder in die Papierbeschichtung (vorzugsweise Polyethylenschichten) inkorporiert werden. Es ist aber ebenso möglich, die Verbindungen in die sensibilisierten Silberhalogenidschichten oder/und die nicht-sensibilisierten Schichten, mit denen das Papier beschichtet ist, einzubringen.

Um das Hintergrundweiss von photographischen Papierbildern weiter zu verbessern, ist es vorteilhaft, optische Aufheller, die vorzugsweise flüssig sind, in einer Gelatineschicht zu dispergieren und diese als eine der Schichten auf den Träger aufzubringen. Diese Möglichkeit bietet folgende Vorteile:

(a) Es müssen nur geringe Mengen an optischem Aufheller eingesetzt werden, da während der Entwicklung keine Verluste auftreten.

(b) Es werden keine unerwünschten Quellen für eine Streuung des Lichts eingeführt.

(c) Es werden hohe Fluoreszenzausbeuten erzielt.

Die erfindungsgemässen Verbindungen der Formel I sind entweder flüssig oder sind sehr leicht löslich bzw. mischbar in typischen hochsiedenden organischen Lösungsmitteln, wie sie in der einschlägigen Industrie, insbesondere der Photoindustrie, eingesetzt werden, und die Verbindungen neigen nicht dazu, in den photographischen Schichten auszukristallisieren.

Sofern Verbindungen der Formel I nicht schon flüssig sind, können durch Mischen von mehreren Verbindungen der Formel I mit niederem Schmelzpunkt oder von flüssigen und festen Verbindungen flüssige Produkte erhalten werden, die bei Raumtemperatur flüssig sind und die oben angegebenen Vorteile aufweisen.

Werden die erfindungsgemässen Aufheller mit Hilfe eines Lösungs (Dispersions)mittels in das entsprechende Material eingearbeitet, kommen als typische Lösungsmittel beispielsweise die folgenden in Betracht: Phthalsäurester (z.B. Dimethyl-, Diethyl-, Dibutyl-, Diamyl-, Dihexyl-, Diheptyl-, Dioctyl-, Dinonyl- oder Didecylphthalat, oder Dibutyl-chlorphthalat), Glycolsäureester (z.B. Butylphthalylbutylglycolat), Phenole (z.B. 2,4-Di-n-amylphenol, 2,4-Di-tert.Amylphenol), Phosphonsäureester (z.B. Diphenyl-, Triphenyl-, Tricresyl-, Cresyl-diphenyl-, Dioctyl-, Dioctyl-butyl-, Trioctyl-, Tridecyl-, Trixylenyl-, Tri(isopropylphenyl)-, Tributyl-, Trihexyl-, Trinonyl-, Trioleyl- oder Tri(butoxyethyl)-phosphat), Citronensäureester (z.B. O-Acetyl-triethyl-, -tributyl-, -trihexyl-, -trioctyl-, -trinonyl-oder -tridecylcitrat), Benzoesäureester (z.B. Butyl-, Hexyl-, Heptyl-, Octyl-, Nonyl-, Decyl-, Undecyl-, Tetradecyl-, Octadecyl- oder Oleyl-benzoat), Ester von substituierten Benzoesäuren (z.B. Butyl-2-methoxybenzoat, Pentyl-o-methylbenzoat, Decyl-p-methylbenzoat, Lauryl-o-chlorbenzoat, Propyl-2,4-dichlorbenzoat, Oleyl-2,4-dichlorbenzoat oder Octyl-p-methoxybenzoat), Fettsäureester und Dicarbonsäureester (z.B. Hexadecyl-myristat, Dibutylsebacat, Dibutoxyethyl-succinat, Dioctyl-adipat, Dioctylazelat, Benzyl-caprylat), Ester von Polyolen (z.B. Decamethylenglykoldiacetat, Triacetyl- oder Tributyroyl-glycerin,

Pentaerythrittetracapronat, oder Isosorbit-di-caprylat), Fettsäureamide (z.B. N,N-Dimethyl-, N,N-Diethyl- oder N,N-Dibutyllaurylamid), chlorierte Paraffine, aliphatische oder aliphatischaromatische Ether (z.B. Glycerin-trialkylether, Glycerin-1,3-dialkylether, n-Pentadecylphenylether oder 3-Pentadecylphenyl-ethylether), Alkylarylcarbamate (z.B. Ethyl-N,N-diphenyl-carbamat) oder Mischungen solcher Flüssigkeiten, sowie auch hochsiedende organische Lösungsmittel,die mindestens ein Chloratom enthalten, insbesondere solche, wie sie in der JP-A 61-124939 beschrieben sind.

Es ist auch möglich, die erfindungsgemässen Verbindungen zusammen mit anderen optischen Aufhellern in der vorstehend beschriebenen Weise einzusetzen, beispielsweise mit anderen Thiophenderivaten, wie sie u.a. in den US-A 3,449,257 und 3,135,762 und in der JP-A 61-124 939 beschrieben sind, aber auch mit optischen Aufhellern anderer Klassen, beispielsweise vom Typ der Oxazole, Stilbene, Cumarine, Thiazole, Imidazole, Imidazolone, Pyrazole oder Naphthalimide, wie sie beispielsweise ebenfalls in der JP-A 61-124 939 erwähnt sind.

Die erfindungsgemässen Verbindungen können z.B. auch in verschiedene Schichten von Photopapieren eingearbeitet werden, in die normalerweise auch UV-Absorber inkorporiert werden, wie es beispielsweise in der EP-A 106690 beschrieben ist. Dabei können Aufheller und UV-Absorber in die gleiche oder in verschiedene Schichten eingearbeitet werden.

Wenn man die erfindungsgemässen Thiophenderivate in eine Gelatineschicht eines Aufzeichnungsmaterials,insbesondere eines Photopapiers, einarbeiten will, sind in der Regel oberflächenaktive Verbindungen zuzusetzen. Als solche kommen zweckmässig nichtionische oder anionische Tenside in Betracht, beispielsweise Alkalimetallsalze von Alkylsulfaten, Alkylarylsulfaten, Alkylarylethersulfaten oder Alkylarylpolyethersulfaten, Alkylbernsteinsäureester usw. Um eine möglichst feine Verteilung der erfindungsgemässen Verbindungen im entsprechenden Medium zu erhalten, wird letzteres zusammen mit den genannten Verbindungen zweckmässig in Geräten wie einem Homogenisator, einer Kolloidmühle, einem Hochgeschwindigkeitsrührwerk oder einem Ultraschallmischer homogenisiert. Zusätzlich kann der Thiophen-Aufheller in einem niedriger siedenden (z.B. unter 150°C), nicht-wassermischbaren Lösungsmittel vorgelöst werden, welches anschliessend vor der Beschichtung des Trägers (z.B. des Papiers) oder während der Beschichtung z.B. durch Verdampfung entfernt wird. Beispiele für solche niedriger siedende Lösungsmittel sind:

Alkylacetate oder -propionate (z.B. Methyl-, Ethyl-, n-Propyl-, Isopropyl- oder Butylacetat, Methyl- oder Ethylpropionat), Ethylformiat, Diethyl-carbonat, niedere Chloralkane (z.B. Tetrachlormethan, Di- und Trichlorethylen, 1,2-Dichlorpropan, Chloroform oder Amylchlorid), Ketone (z.B. Aceton, Methyl-ethyl-keton, Diethylketon oder Methyl-isobutylketon), Ether (z.B. Diisopropylether, Dibutylether, Tetrahydrofuran, Dioxan), Alkohole (z.B. Methanol, Ethanol, Isopropanol, Butanol), Monoether von Diolen (z.B. Ethylenglykol-monomethylether oder -monoethylether), Kohlenwasserstoffe (z.B. Cyclohexan, Methylcyclohexan, Ligroin, Benzol, Toluol, Xylol), Nitromethan, Acetonitril, Dimethylsulfoxid, N-Methylpyrrolidon, Dimethylformamid, Tetrahydrothiophen-dioxid, Butyrolacton oder 1,2-Dimethoxyethan.

Die erfindungsgemässen Thiophenverbindungen können auch in photographischen Systemen angewendet werden, die einen hydrophoben Latexträger aufweisen, in welchem die Thiophenverbindungen entweder in gelöstem Zustand oder in den Latexteilchen eingeschlossen vorliegen können. Beispiele von solchen Latices und deren allgemeine Verwendung zusammen mit optischen Aufhellern sind z.B. in den US-A 4,608,424, EP-A 69 671 und 146 337 enthalten.

Wie bereits angedeutet, können die erfindungsgemässen optischen Aufheller in jede beliebige Schicht eines Bilderzeugungs- oder Aufzeichnungsmaterials inkorporiert werden. Sie können allein in einer gesonderten Schicht oder aber in Schichten enthalten sein, die weitere Wirkstoffe, beispielsweise UV-Absorber, Farbkuppler, Fänger ("scavenger") für oxidierte Entwicklersubstanz oder/und Silberhalogenid beinhalten. Bevorzugt befinden sie sich jedoch in einer oder mehreren Schichten, die oberhalb der UV-Absorber enthaltenden Schicht(en) angeordnet sind, da sich dann die Wirksamkeit des optischen Aufhellers besser entfalten kann.

Erfindungsgemässe Thiophenderivate enthaltende photographische Materialien müssen nicht in besonderen Bädern entwickelt werden. Es kann sich jedoch als zweckmässig erweisen, dem Entwicklerbad wasserlösliche fluoreszierende Verbindungen zuzusetzen, besonders weil solche Verbindungen den von Sensibilisierungsfarbstoffen erzeugten Schleier reduzieren. Beispiele für solche wasserlösliche fluoreszierende Verbindungen, wie sie zusammen mit den erfindungemässen Thiophenderivaten verwendet werden können, sind solche, wie sie in der US-A 4,587,195 beschrieben sind, insbesondere der Formel

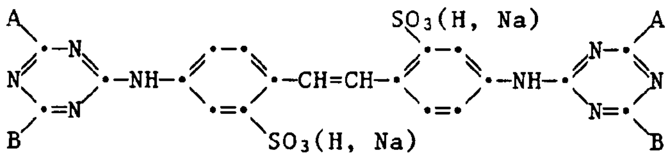

worin A Morpholino, Dimethylamino, Diethylamino oder Methoxy und B $-NHCH_2CH_2SO_3(H, Na)$, $-N(CH_2CH_2OH)_2$ oder

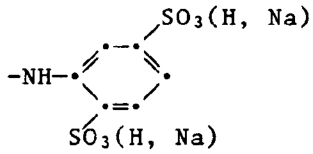

bedeuten.

Die erfindungsgemässen optischen Aufheller können ferner auch in Röntgenfilmen, z.B. für medizinische Zwecke, Anwendung finden. Sie eignen sich z.B. vorzüglich für die Einarbeitung zusammen mit einem oder mehreren blauen Farbstoffen in den Polyester-Träger solcher Filme und bewirken einen klareren Blauton des Films. Sie können aber auch in eine oder mehrere Schichten des Films eingearbeitet werden, wobei die bereits beschriebenen Methoden angewendet werden können. Auch die Einarbeitung in andere Arten von photographischen Filmen, z.B. für Transparentmaterialien, nach den erwähnten Methoden ist möglich, wenn eine blaue Fluoreszenz erwünscht ist.

Die erfindungsgemässen Thiophenderivate können analog den vorstehend beschriebenen Methoden auch in beliebige andere photographische Materialien inkorporiert werden, beispielsweise in Photosatzpapiere, photographische Bilder erhalten nach dem Silberfarbbleichverfahren, dem Silberfarbdiffusionstransfer-Verfahren, der beizenden Entwicklung, dem Silbersalzdiffusionsverfahren oder dem Wärmeentwicklungs- oder "Trockensilber"-Verfahren.

Die Verwendung der erfindungsgemässen Thiophen-Aufheller ist im Fall von Aufhellung von Abbildungs- und Aufzeichnungsmaterialien keineswegs auf durch Silberhalogenid sensibilisierte Materialien (z.B. Papier) beschränkt. Sie können ebenso in Druck- und farbbildenden Systemen eingesetzt werden, die Bilderzeugungs- und Aufzeichnungstechniken verwenden, die auf Photopolymerisation, Photoplastifizierung (photoplasticisation), hitze- oder lichtempfindlichen Diazoniumsalzen, Leukofarbstoffen und Oxidationsmitteln, Farbstoffdiffusion in der Wärme, Farbbildnerdiffusion in der Wärme, Farbstofflactonen und Lewis-Säure, oder dem Aufbrechen von Farbstoffe oder farblose Farbbildner enthaltenden Mikrokapseln, beruhen. Weiter können die Verbindungen der Formel I in Aufzeichnungsmaterialien enthalten sein, die mit elektrostatischen, elektrophotographischen, elektrophoretischen, magnetographischen oder laserelektrophotographischen Druckern bzw. Tintenstrahl-, thermischen Tintenstrahl ("bubble-jet")-, Thermotransfer- oder Punktmatrix-Druckern oder Feder-Plottern verwendet werden können.

Ein weiterer Gegenstand der Erfindung sind Abbildungs- und Aufzeichnungsmaterialien, die mindestens eine Verbindung der Formel I enthalten.

Die Menge an optischen Aufhellern der Formel I, die den jeweiligen Substraten zugegeben werden, kann je nach Anwendungsbereich in weiten Grenzen schwanken. In den genannten Abbildungs- und Aufzeichnungsmaterialien können sie beispielsweise in einer Menge von 1-500, insbesondere 2-250, vorzugsweise 5-50 mg pro Quadratmeter der genannten Materialien zugesetzt weden.

Die Verbindungen der Formel I können nach an sich bekannten Verfahren hergestellt werden. Beispiele für solche Verfahren sind in den US-A 3,136,773 und 4,267,343 angegeben.

Beispielsweise setzt man etwa ein Moläquivalent einer Thiophendicarbonsäure der Formel IV

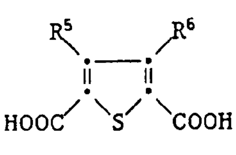

(IV)

oder ein funktionelles Derivat davon mit insgesamt etwa 2 Moläquivalenten o-Aminophenol der Formel V

oder/und Va

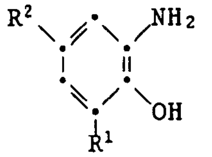
(V)

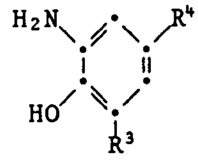
(Va)

gegebenenfalls in Gegenwart eines Katalysators bei erhöhter Temperatur um, wobei das entstehende Zwischenprodukt der Formel VI

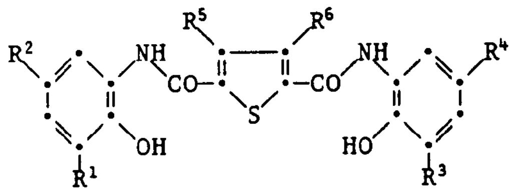
(VI)

entweder isoliert werden kann oder die Reaktion als Eintopfverfahren direkt ohne Isolierung des Zwischenprodukts bis zum Endprodukt der Formel I weitergeführt wird.

Als funktionelle Derivate der Dicarbonsäuren der Formel IV werden insbesondere deren niedrige Alkylester und vor allem deren Halogenide, insbesondere Chloride, verwendet.

Wird das Verfahren einstufig ausgeführt, beträgt die Reaktionstemperatur z.B. 120 - 260°C, insbesondere 160 - 260°C, und die Reaktion wird vorteilhaft unter Inertgasatmosphäre, z.B. unter Stickstoff, durchgeführt. Zweckmässig wird ein Katalysator, insbesondere ein Säurekatalysator, zugegeben, z.B. Borsäure, Zinkchlorid, p-Toluolsulfonsäure oder Polyphosphorsäuren einschliesslich Pyrophosphorsäure. Die Menge an Katalysator beträgt z.B. 0,5 - 5 %, bezogen auf das Gewicht des Reaktionsgemisches. Die Reaktion kann in der Schmelze oder unter Verwendung eines Lösungsmittels durchgeführt werden. Beispiele für solche Lösungsmittel sind Dimethylformamid und gegebenenfalls veretherte Hydroxyverbindungen wie z.B. Propylenglykol, Ethylenglykol-monoethylether oder Diethylenglykoldiethylether.

Wird das Verfahren zweistufig durchgeführt, kann die erste Stufe vorteilhaft in Gegenwart eines organischen Lösungsmittels, beispielsweise eines gegebenenfalls Halogen- oder Nitro-substituierten aromatischen Kohlenwasserstoffs, wie z.B. von Benzol, Toluol, Xylol, Chlorbenzol, Dichlorbenzol oder Nitrobenzol, ausgeführt werden. Die Reaktionstemperatur kann dabei z.B. 40 - 150°C, insbesondere 50 - 120°C betragen. Vorteilhaft erhitzt man das Reaktionsgemisch auf die Rückflusstemperatur des verwendeten Lösungsmittels. Die zweite Stufe (Ringschlussreaktion) wird zweckmässig unter den für das Einstufenverfahren beschriebenen bevorzugten Reaktionsbedingungen durchgeführt.

Nähere Einzelheiten über das beschriebene Herstellungsverfahren sind der US-A 3,136,773 sowie den nachfolgenden Beispielen zu entnehmen.

Manche Verbindungen der Formel I können auch dadurch hergestellt werden, dass man nach dem vorstehend beschriebenen Verfahren erhaltene Verbindungen in andere Verbindungen der genannten Formel umwandelt. So kann man, ausgehend von erhaltenen Verbindungen mit X = -COOH in den Substituenten $R^1$ - $R^4$ durch übliche Veresterung oder Salzbildung Verbindungen mit X = -COOR$^9$ mit $R^9 \neq H$ oder durch Umsetzung mit einem Amin HNR$^{12}$R$^{13}$ (vorteilhaft nach vorgängiger Ueberführung in das Säurechlorid) Verbindungen mit X = -CONR$^{12}$R$^{13}$ herstellen. Umgekehrt können aus erhaltenen Estern (X = -COOR$^9$ mit $R^9 \neq H$, Kation) oder Amiden (X = -CONR$^{12}$R$^{13}$) durch Verseifung die freie Säure (X = -COOH) oder durch Umesterung aus den genannten Estern andere Ester bzw. durch Umamidierung aus den genannten Amiden andere Amide hergestellt werden. Die entsprechenden Verfahren sind dem Fachmann geläufig.

Die Ausgangsverbindungen der Formel (V) bzw. (Va) sind bekannt oder lassen sich nach an sich bekannten Verfahren herstellen. Man erhält sie beispielsweise, indem man ein Phenol der Formel VII bzw. VIIa

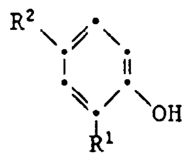

(VII)

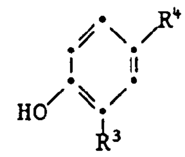

(VIIa)

mit einem Diazoniumsalz (beispielsweise dem Diazoniumsalz aus Anilin) kuppelt und die erhaltene Azoverbindung reduktiv (z.B. mit Natriumhydrogensulfit) zum entsprechenden o-Aminophenol spaltet. Alternativ kann man ein Phenol der Formel VII oder VIIa nach üblichen Nitrierverfahren nitrieren und das erhaltene o-Nitrophenol der Formel VIII bzw. VIIIa

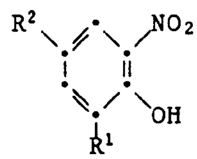

(VIII)

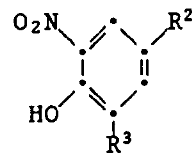

(VIIIa)

nach üblichen Methoden, z.B. durch katalytische Hydrierung, zum entsprechenden o-Aminophenol der Formel V bzw. Va reduzieren.

Phenole der Formel VII bzw. VIIa sind bekannt bzw. können nach bekannten Verfahren hergestellt werden. Siehe dazu z.B. die EP-A 106799. o-Nitrophenole der Formel VIII bzw. VIIIa und deren Herstellung sind auch beispielsweise in der EP-A 323408 beschrieben.

Die Thiophendicarbonsäuren der Formel IV und deren funktionelle Derivate sind bekannt und können nach bekannten Verfahren hergestellt werden. Siehe z.B. US-A 3,136,773.

Eine weitere Möglichkeit, die erfindungsgemässen Verbindungen herzustellen, besteht in der Umsetzung eines Sulfids der Formel IX

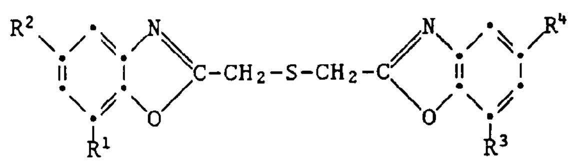 (IX)

mit einer Dicarbonylverbindung der Formel X

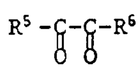 (X)

oder einem Derivat davon.

Die Verbindungen der Formel IX erhält man

a) durch Umsetzung einer Verbindung der Formel XI

$$X - CO - CH_2 - S - CH_2 - CO - X \quad (XI)$$

mit 2 Moläquivalenten o-Aminophenol der Formel V oder/und Va,

b) durch Umsetzung einer Verbindung der Formel XII oder/und XIIa

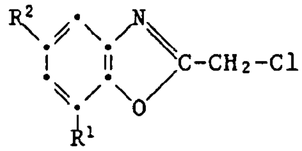

oder/und

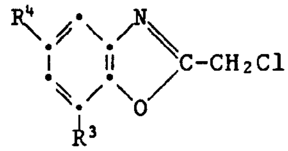

(XII) (XIIa)

mit Natriumsulfid oder

c) durch Umsetzung einer Verbindung der Formel XIII bzw. XIIIa

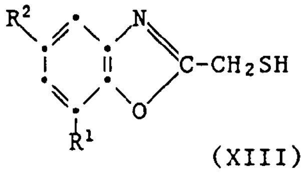

(XIII)

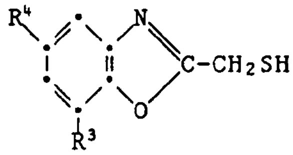

(XIIIa)

mit einer Verbindung der Formel XII bzw. XIIa.

Die Verbindungen der Formeln XII und XIII bzw. XIIa und XIIIa können beispielsweise hergestellt werden

(1) durch Umsetzung von Chloressigsäure oder Thioglykolsäurechlorid mit Aminophenolen der Formel V bzw. Va oder

(2) durch Umsetzung von Chlorhydraten von 2-Chlor- oder 2-Mercaptoacetimidoalkylethern mit Aminophenolen der Formel V bzw. Va.

Einzelheiten zu dem vorstehend beschriebenen Verfahren sind der US-A 4,267,343 zu entnehmen.

Schliesslich können die Verbindungen der Formel I auch nach dem Reaktionsschema

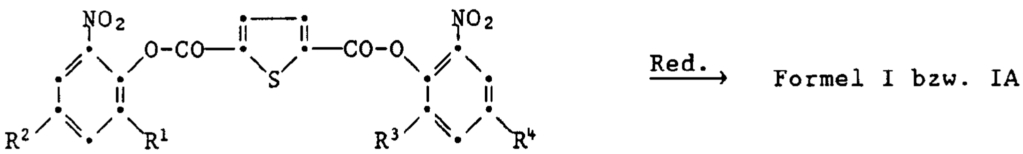


hergestellt werden. Red. bedeutet dabei ein Reduktionsmittel, z.B. Cu.

Die nachfolgenden Beispiele erläutern die Erfindung weiter. In den Beispielen ebenso wie in der übrigen Beschreibung und in den Patentansprüchen bedeuten Teile und Prozentangaben Gewichtsteile und Gewichtsprozent, sofern nichts anderes angegeben ist.

Beispiel 1:

1891 g 3-(3-t-Butyl-4-hydroxyphenyl)-propionsäuremethylester werden in 2700 ml Toluol gelöst und der Lösung wird 1 g Natriumnitrit zugefügt. Danach wird eine Mischung aus 1008 g 67 %iger wässriger Salpetersäure und 1000 ml Wasser langsam zugegeben, wobei die Reaktionstemperatur bei 5 - 10°C gehalten wird. Die Reaktionsmischung wird dann noch 18 Stunden bei Raumtemperatur gerührt. Danach wird die organische Phase abgetrennt, mit $NaHCO_3$-Lösung und Wasser gewaschen und eingeengt. Der Rückstand wird unter Hochvakuum destilliert, wobei das Produkt bei 153-160°C bei $10^{-1}$ mmHg übergeht. Man erhält so 1509 g 3-(3-t-Butyl-4-hydroxy-5-nitrophenyl)-propionsäuremethylester als gelben Feststoff mit einem Schmelzpunkt von 41 - 42°C.

In analoger Weise werden die in Tabelle 1 angegebenen Nitrophenole der Formel

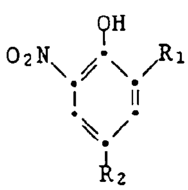

erhalten.

Tabelle 1

| Beispiel Nr. | $R_1$ | $R_2$ | |
|---|---|---|---|
| 2 | $-CH_3$ | $-CH_2CH_2COOCH_3$ | Fp= 77°C |
| 3 | $-C(CH_3)_2(CH_2)_3COOCH_3$ | $-C(CH_3)_3$ | Oel |
| 4 | $-C(CH_3)_2(CH_2)_3COOCH_3$ | $-C(CH_3)_2(CH_2)_3COOCH_3$ | Oel |
| 5 | $-C(CH_3)_3$ | $-COOCH_3$ | Fp= 116°C |
| 6 | $-C(CH_3)_3$ | $-CH_2CH(CH_3)COOCH_3$ | Oel |
| 7 | $-C(CH_3)_2(CH_2)_3COOCH_3$ | $-C_2H_5$ | Oel |
| 8 | $-C_2H_5$ | $-C(CH_3)_2(CH_2)_3COOCH_3$ | Oel |
| 9 | $-CH(CH_3)_2$ | $-C(CH_3)_2(CH_2)_3COOCH_3$ | Oel |
| 10 | $-CH(CH_3)C_2H_5$ | $-C(CH_3)_2(CH_2)_3COOCH_3$ | Oel |
| 11 | $-C(CH_3)_3$ | $-CH(COOCH_3)CH_2COOCH_3$ | Oel |
| 12 | $-C(CH_3)_3$ | $-CH_2CH(COOCH_3)_2$ | Oel |

Beispiel 13:

1597 g 3-(3-t-Butyl-4-hydroxy-5-nitrophenyl)-propionsäuremethylester (erhalten z.B. gemäss Beispiel 1) werden in 16 l Ethylacetat gelöst. Die Lösung wird 23 Stunden bei einem $H_2$-Druck von 5 bar und unter Verwendung eines Raney-Nickel-Katalysators hydriert. Der Katalysator wird unter $N_2$-Atmosphäre abfiltriert und das Filtrat eingeengt. Man erhält als Rückstand 1299 g 3-(3-t-Butyl-4-hydroxy-5-aminophenyl)-propionsäuremethylester mit einem Schmelzpunkt von 108-109°C.

In analoger Weise werden die in Tabelle 2 angegebenen Aminophenole der Formel

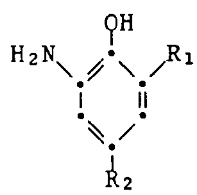

erhalten.

Tabelle 2

| Beispiel Nr. | $R_1$ | $R_2$ | |
|---|---|---|---|
| 14 | $-CH_3$ | $-CH_2CH_2COOCH_3$ | Fp= 84°C |
| 15 | $-C(CH_3)_2(CH_2)_3COOCH_3$ | $-C(CH_3)_3$ | a) |
| 16 | $-C(CH_3)_2(CH_2)_3COOCH_3$ | $-C(CH_3)_2(CH_2)_3COOCH_3$ | Oel |
| 17 | $-C(CH_3)_3$ | $-COOCH_3$ | Fp= 121°C |
| 18 | $-C(CH_3)_3$ | $-CH_2CH(CH_3)COOCH_3$ | Oel |
| 19 | $-C(CH_3)_2(CH_2)_3COOCH_3$ | $-C_2H_5$ | Oel |
| 20 | $-C_2H_5$ | $-C(CH_3)_2(CH_2)_3COOCH_3$ | Oel |
| 21 | $-CH(CH_3)_2$ | $-C(CH_3)_2(CH_2)_3COOCH_3$ | Oel |
| 22 | $-CH(CH_3)C_2H_5$ | $-C(CH_3)_2(CH_2)_3COOCH_3$ | Oel |
| 23 | $-C(CH_3)_3$ | $-CH(COOCH_3)CH_2COOCH_3$ | Oel |
| 24 | $-C(CH_3)_3$ | $-CH_2CH(COOCH_3)_2$ | Oel |

a) Das Produkt wird nicht isoliert, sondern direkt in der nächsten Stufe eingesetzt.

Beispiel 25:

68,8g Thiophen-2,5-dicarbonsäure werden in 400 ml Toluol dispergiert und 4 ml Dimethylformamid zugegeben. 69,6 ml Thionylchlorid werden bei 70°C zugetropft. Unter Stickstoffatmosphäre wird die erhaltene Mischung 5 Stunden unter Rückfluss gehalten. Dann wird überschüssiges Thionylchlorid abdestilliert und 132,5 g 3-(3-t-Butyl-4-hydroxy-5-aminophenyl)-propionsäure-methylester werden zugefügt. Man stellt HCl-Entwicklung und einen gelben Niederschlag fest. Das Reaktionsgemisch wird noch 4 Stunden bei 80°C unter Stickstoff gerührt und dann abkühlen gelassen. Der Niederschlag wird abfiltriert, mit Toluol gewaschen und im Vakuum getrocknet. Man erhält so 212 g 2,5-Bis[3-t-butyl-2-hydroxy-5-(2-methoxycarbonyl-1-ethyl)phenylaminocarbonyl]thiophen der Formel

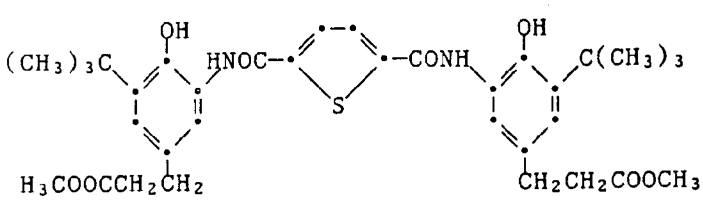

als gelbliches Pulver mit einem Schmelzpunkt von 172-173°C.

In analoger Weise weden die in Tabelle 3 angegebenen Thiophen-bis-carbonsäureamide der Formel

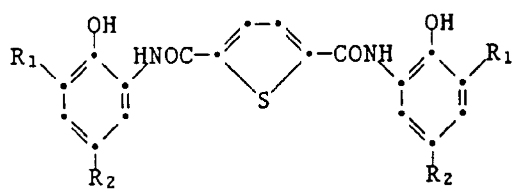

erhalten.

Tabelle 3

| Beispiel Nr. | $R_1$ | $R_2$ | Schmelzpunkt (°C) |
|---|---|---|---|
| 26 | $-CH_3$ | $-CH_2CH_2COOCH_3$ | 190-195 |
| 27 | $-C(CH_3)_2(CH_2)_3COOCH_3$ | $-C(CH_3)_3$ | 143-144 |
| 28 | $-C(CH_3)_3$ | $-COOCH_3$ | 208-209 |
| 29 | $-C(CH_3)_3$ | $-CH_2CH(CH_3)COOCH_3$ | 93-95 |
| 30 | $-C(CH_3)_2(CH_2)_3COOCH_3$ | $-C_2H_5$ | 138-139 |
| 31 | $-C_2H_5$ | $-C(CH_3)_2(CH_2)_3COOCH_3$ | 140-141 |
| 32 | $-CH(CH_3)_2$ | $-C(CH_3)_2(CH_2)_3COOCH_3$ | 62-63 |
| 33 | $-CH(CH_3)C_2H_5$ | $-C(CH_3)_2(CH_2)_3COOCH_3$ | Oel |
| 34 | $-C(CH_3)_3$ | $-CH(COOCH_3)CH_2COOCH_3$ | 110-111 |
| 35 | $-C(CH_3)_3$ | $-CH_2CH(COOCH_3)_2$ | Harz |

Beispiel 36:

89,3 g 2,5-Bis-[3-t-butyl-2-hydroxy-5-(2-methoxycarbonyl-1-ethyl)-phenylaminocarbonyl]-thiophen werden mit 1 g Borsäure verrieben und unter Argonatmosphäre auf 230°C erhitzt, wobei das entstehende Reaktionswasser abdestilliert wird. Nach 6 Stunden bei dieser Temperatur destilliert kein Wasser mehr ab. Der hellbraune Rückstand wird in Ethanol aufgenommen und das Produkt auskristallisieren gelassen. Nach Filtration und Trocknung erhält man 2 g 2,5-Bis-[7-t-butyl-5-(2-methoxycarbonyl-1-ethyl)-benzoxazol-2-yl]-thiophen der Formel

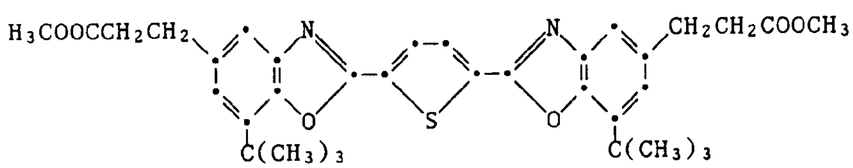

in Form gelber Kristalle mit einem Schmelzpunkt von 144-145°C.

In analoger Weise werden die in Tabelle 4 angegebenen Bis-benzoxazolylthiophene der Formel

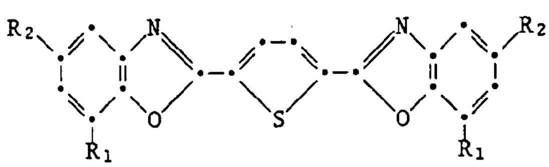

erhalten.

Tabelle 4

| Beispiel Nr. | $R_1$ | $R_2$ | Schmelzpunkt (°C) |
|---|---|---|---|
| 37 | $-CH_3$ | $-CH_2CH_2COOCH_3$ | 139-141 |
| 38 | $-C(CH_3)_2(CH_2)_3COOCH_3$ | $-C(CH_3)_3$ | 63-66 |
| 39 | $-C(CH_3)_3$ | $-COOCH_3$ | 209-210 |
| 40 | $-C(CH_3)_2(CH_2)_3COOCH_3$ | $-C(CH_3)_2(CH_2)_3COOCH_3$ | Oel |
| 41 | $-C(CH_3)_3$ | $-CH_2CH(CH_3)COOCH_3$ | 132-133 |
| 42 | $-C(CH_3)_2(CH_2)_3COOCH_3$ | $-C_2H_5$ | 72-73 |
| 43 | $-C_2H_5$ | $-C(CH_3)_2(CH_2)_3COOCH_3$ | Oel |
| 44 | $-CH(CH_3)_2$ | $-C(CH_3)_2(CH_2)_3COOCH_3$ | Oel |
| 45 | $-CH(CH_3)C_2H_5$ | $-C(CH_3)_2(CH_2)_3COOCH_3$ | 74-75 |
| 46 | $-C(CH_3)_3$ | $-CH(COOCH_3)CH_2COOCH_3$ | 111-112 |
| 47 | $-C(CH_3)_3$ | $-CH_2CH(COOCH_3)_2$ | 95-97 |

Beispiel 48:

10 g 2,5-Bis[7-t-butyl-5-(2-methoxycarbonyl-1-ethyl)-benzoxazol-2-yl]-thiophen (siehe Beispiel 36), 40 ml n-Octan-1-ol und 0,3 g Dibutylzinnoxid werden unter Rühren auf 120°C erhitzt und bei dieser Temperatur 5 Stunden weitergerührt. Das entstehende Methanol wird abdestilliert. Die Reaktionsmischung wird über ein Filterhilfsmittel (®Prolith Rapid) filtriert, welches dann mit $CH_2Cl_2$ gewaschen wird. Das Filtrat wird eingeengt und das überschüssige 1-Octanol im Vakuum abdestilliert. Der Rückstand wird aus Methanol umkristallisiert. Man erhält so 7,7 g 2,5-Bis-[7-t-butyl-5-(2-n-octyloxycarbonyl-1-ethyl)-benzoxazol-2-yl]-thiophen der Formel

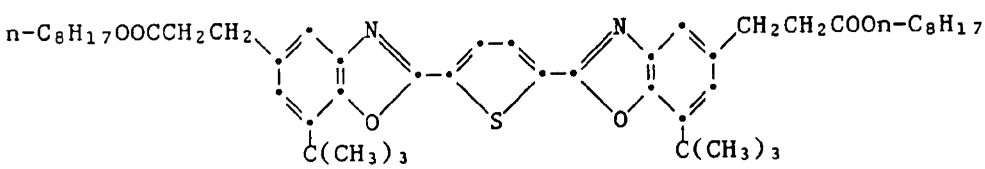

in Form gelber Kristalle mit einem Schmelzpunkt von 68 - 70°C.

In analoger Weise werden die in Tabelle 5 angegebenen Bis-benzoxazolylthiophene der Formel

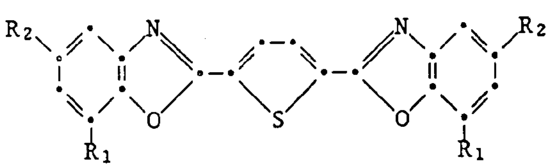

erhalten.

Tabelle 5

| Beispiel Nr. | $R_1$ | $R_2$ | Schmelzpunkt (°C) |
|---|---|---|---|
| 49 | $-CH_3$ | $-CH_2CH_2COOCH_2CH(C_2H_5)(CH_2)_3CH_3$ | Harz |
| 50 | $-CH_3$ | $-CH_2CH_2COO-n-C_8H_{17}$ | 71-74 |
| 51 | $-C(CH_3)_3$ | $-COOCH_2CH(C_2H_5)(CH_2)_3CH_3$ | 134-136 |
| 52 | $-C(CH_3)_3$ | $-COO-n-C_8H_{17}$ | 172-173 |
| 53 | $-C(CH_3)_3$ | $-COOC_8H_{17}$ (Isomerengemisch) | 70 |
| 54 | $-C(CH_3)_3$ | $-CH_2CH_2COOCH_2CH(C_2H_5)(CH_2)_3CH_3$ | 56-58 |
| 55 | $-C(CH_3)_3$ | $-CH_2CH_2COOCH_2CH(CH_3)C_2H_5$ | 82-83 |
| 56 | $-C(CH_3)_3$ | $-CH_2CH_2COOCH_2CH(CH_3)(CH_2)_2CH_3$ | 57-58 |
| 57 | $-C(CH_3)_3$ | $-CH_2CH_2COOCH_2CH_2OCH_2CH_2OC_2H_5$ | Oel |
| 58 | $-C(CH_3)_3$ | $-CH_2CH_2COOCH_2CH_2OCH_2CH_2O(CH_2)_3CH_3$ | Oel |
| 59 | $-C(CH_3)_3$ | $-CH_2CH_2COOCH_2-$ 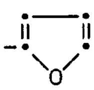 | 144-146 |
| 60 | $-C(CH_3)_3$ | $-CH_2CH_2COOCH_2CH_2P[O(CH_2)_3CH_3]_2$<br>O | Oel |
| 61 | $-C(CH_3)_3$ | $-COOCH_2CH_2P(OCH_3)_2$<br>O | |
| 62 | $-C(CH_3)_3$ | $-CH_2CH(CH_3)COOCH_2CH(C_2H_5)(CH_2)_3CH_3$ | Oel |
| 63 | $-C(CH_3)_3$ | $-CH_2CH(CH_3)COOCH_2CH(CH_3)(CH_2)_2CH_3$ | Oel |
| 64 | $-C_2H_5$ | $-C(CH_3)_2(CH_2)_3COOCH_2CH(C_2H_5)(CH_2)_3CH_3$ | Oel |
| 65 | $-C_2H_5$ | $-C(CH_3)_2(CH_2)_3COOCH_2CH(CH_3)(CH_2)_2CH_3$ | Oel |
| 66 | $-CH(CH_3)_2$ | $-C(CH_3)_2(CH_2)_3COOCH_2CH(CH_3)(CH_2)_2CH_3$ | Oel |
| 67 | $-CH(CH_3)C_2H_5$ | $-C(CH_3)_2(CH_2)_3COOCH_2CH(C_2H_5)(CH_2)_3CH_3$ | Oel |
| 68 | $-C(CH_3)_3$ | $-CHCH_2COOCH_2CH(CH_3)(CH_2)_2CH_3$<br>$COOCH_2CH(CH_3)(CH_2)_2CH_3$ | Oel |

Tabelle 5 (Fortsetzung)

| Beispiel Nr. | $R_1$ | $R_2$ | Schmelz-punkt (°C) |
|---|---|---|---|
| 69 | $-C(CH_3)_3$ | $-CH_2CH[COOCH_2CH(CH_3)(CH_2)_2CH_3]_2$ | Oel |
| 70 | $-C(CH_3)_2(CH_2)_3COOCH_2CH(C_2H_5)(CH_2)_3CH_3$ | $-C_2H_5$ | Oel |
| 71 | $-C(CH_3)_2(CH_2)_3COOCH_2CH(CH_3)(CH_2)_2CH_3$ | $-C(CH_3)_2(CH_2)_3COOCH_2CH(CH_3)(CH_2)_2CH_3$ | Oel |
| 72 | $-C(CH_3)_3$ | $-CH_2CH_2COOCH_2CH_2CH(CH_3)CH_2C(CH_3)_3$ | <30 |
| 73 | $-C(CH_3)_3$ | $-CH_2CH_2COO(CH_2)_8CH=CH(CH_2)_7CH_3$ Die Verbindung enthält in geringen Mengen auch Anteile, worin die Doppelbindung an anderen Positionen lokalisiert ist. | Oel |
| 74 | $-C(CH_3)_3$ | $CH_2CH_2COOC_8H_{17}$ (Isomerengemisch) | Oel |

Beispiel 75:

224,4 g KOH werden in 1200 ml Methanol gelöst. Man fügt 229 g 2,5-Bis[7-t-butyl-5-(2-methoxycarbonyl-1-ethyl)-benzoxazol-2-yl]-thiophen zu und rührt 2 Stunden lang unter Rückfluss. Nach dem Abkühlen fällt das Bis-Natriumsalz der gebildeten Säure aus. Man filtriert ab und die Mutterlauge wird eingedampft. Die vereinigten Feststoffe werden in 3 l Wasser dispergiert und mit 250 ml konzentrierter HCl angesäuert. Man filtriert den Feststoff ab und wäscht mit 5 l Wasser netural. Das so erhaltene Rohprodukt wird aus 2 l siedendem Ethanol umkristallisiert und bei 100°C im Vakuum getrocknet. Man erhält so 200 g 2,5-Bis-[7-t-butyl-5-(2-carboxy-1-ethyl)-benzoxazol-2-yl]-thiophen der Formel

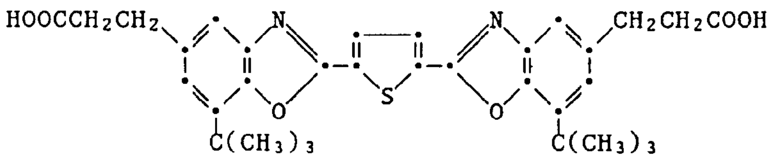

mit einem Schmelzpunkt von 247-248°C.

Beispiel 76:

14,80 g 2,5-Bis-[7-t-butyl-5-(2-carboxy-1-ethyl)-benzoxazol-2-yl]thiophen werden mit 30,0 g Thionylchlorid unter gutem Rühren auf 80°C erwärmt, bis die Chlowasserstoffentwicklung beendet ist. Das überschüssige Thionylchlorid wird unter Vakuum entfernt und der Rückstand 2,5-Bis-[7-t-butyl-5-(2-chlorocarbonyl-1-ethyl)-benzoxazol-2-yl]-thiophen wird in 300 ml trockenem Toluol bei 50°C gelöst. Bei dieser Temperatur werden gleichzeitig 12,0 g Bis-(2-ethylhexyl)amin und 5,0 g Triethylamin während 15 Minuten tropfenweise zugegeben. Das Reaktionsgemisch wird 2 Stunden bei 50°C weitergerührt, abgekühlt, mit verdünnter Salzsäure und Wasser gewaschen, getrocknet und das Lösungsmittel unter erniedrigtem Druck entfernt. Der Rückstand wird über Kieselgel chromatographiert. So erhält man 19,5 g reines 2,5-Bis-{7-t-butyl-5-[2-(N,N-di-2-ethylhexylamido)-1-ethyl]-benzoxazol-2-yl}-thiophen der Formel

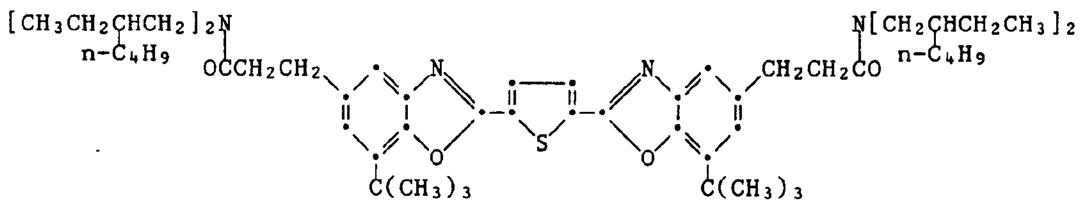

als dickflüssiges Oel.

Beispiel 77:

Verfährt man wie in Beispiel 76 beschrieben und setzt an Stelle des Bis-(2-ethylhexyl)-amins 2-ethylhexylamin ein, erhält man 2,5-Bis-{7-t-butyl-5-[2-(N-2-ethylhexylamido)-1-ethyl]-benzoxazol-2-yl}-thiophen der Formel

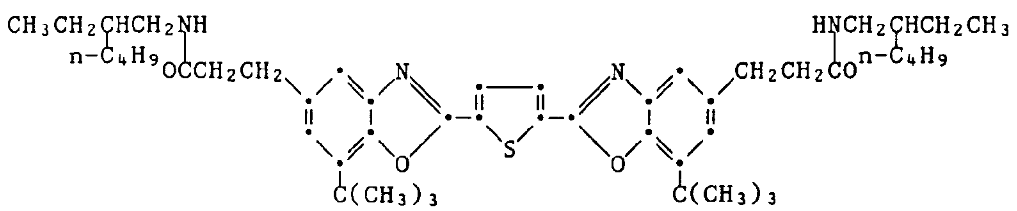

mit einem Schmelzpunkt von 175°-176°C.

Werden im Umesterungsverfahren gemäss Beispiel 48 Mischungen von Alkoholen eingesetzt, so erhält man üblicherweise flüssige Gemische von Verbindungen der Formel I.

Beispiel 78:

Wird an Stelle des n-Octan-1-ols in Beispiel 48 eine 1:1-Mischung von n-Octan-1-ol und 2-Ethylhexan-1-ol eingesetzt, so erhält man ein Isomerengemisch von Verbindungen der Formel

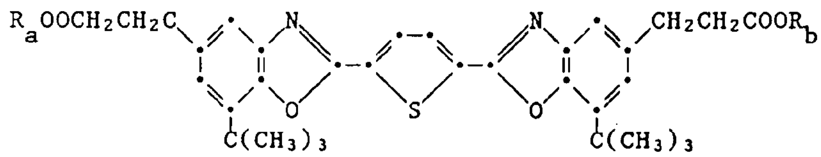

aus den 3 Komponenten
(1) $R_a$ = n-$C_8H_{17}$ , $R_b$ = n-$C_8H_{17}$
(2) $R_a$ = n-$C_8H_{17}$ , $R_b$ = 2-Ethylhexyl
(3) $R_a$ = 2-Ethylhexyl, $R_b$ = 2-Ethylhexyl
im ungefähren Mengenverhältnis 1:2:1.

Die Mischung ist eine ölige Flüssigkeit.

Beispiel 79:

Verwendet man an Stelle von n-Octan-1-ol in Beispiel 48 eine 1 : 1-Mischung aus 2-Ethylhexan-1-ol und 2-Methylpentan-1-ol, so erhält man ein Gemisch von Verbindungen der in Beispiel 78 angegebenen Formel aus den 3 Komponenten
(1) $R_a$ = 2-Ethylhexyl, $R_b$ = 2-Ethylhexyl
(2) $R_a$ = 2-Ethylhexyl, $R_b$ = 2-Methylpentyl
(3) $R_a$ = 2-Methylpentyl, $R_b$ = 2-Methylpentyl
im ungefähren Mengenverhältnis 1:2:1

Die Mischung ist eine ölige Flüssigkeit.

Beispiel 80:

Verwendet man an Stelle von n-Octan-1-ol in Beispiel 48 eine 1:1 Mischung aus Diethylenglykol-monoethylether und Diethylenglykol-mono-n-butylether, so erhält man ein Gemisch von Verbindungen der in Beispiel 78 angegebenen Formel aus den 3 Komponenten
(1) $R_a$ = $-CH_2CH_2OCH_2CH_2OC_2H_5$ $R_b$ = $-CH_2CH_2OCH_2CH_2OC_2H_5$
(2) $R_a$ = $-CH_2CH_2OCH_2CH_2OC_2H_5$ $R_b$ = $-CH_2CH_2OCH_2CH_2O$-n-$C_4H_9$
(3) $R_a$ = $-CH_2CH_2OCH_2CH_2O$-n-$C_4H_9$ $R_b$ = $-CH_2CH_2OCH_2CH_2O$-n-$C_4H_9$
im ungefähren Mengenverhältnis 1:2:1.

Die Mischung ist eine ölige Flüssigkeit.

Beispiel 81:

Verwendet man an Stelle von n-Octan-1-ol in Beispiel 48 eine 1:1 Mischung aus den beiden Alkoholen $HOCH_2CH_2CH(CH_3)CH_2C(CH_3)_3$ und $HOCH_2CH(C_2H_5)$-n-$C_4H_9$, so erhält man ein Gemisch von Verbindungen der in Beispiel 78 angegebenen Formel aus den 3 Komponenten
(1) $R_a$ = $R_b$ = $-CH_2CH_2CH(CH_3)CH_2C(CH_3)_3$
(2) $R_a$ = $-CH_2CH_2CH(CH_3)CH_2C(CH_3)_3$ $R_b$ = $-CH_2CH(C_2H_5)$-n-$C_4H_9$
(3) $R_a$ = $R_b$ = $-CH_2CH(C_2H_5)$-n-$C_4H_9$
im ungefähren Verhältnis 1:2:1. Die Mischung ist eine ölige Flüssigkeit.

Beispiel 82:

Herstellung einer Gelatineschicht enthaltend erfindungsgemässe Thiophenderivate

a) Folgende Komponenten werden mit Ultraschall 3 Minuten lang emulgiert:

| | |
|---|---|
| 1250 mg | Gelatine |
| 510 mg | Trikresylphosphat |
| 100 mg | 4,8-Diisobutylnaphthalin-2-sulfonsäure-Na-Salz (Tensid) |
| 80 mg | Härter (2-Hydroxy-4,6-dichlor-1,3,5-triazin) |
| 700 mg | Essigsäureethylester |
| 0,4 mMol | der zu prüfenden Verbindung der Formel I |

ad 24 ml deionisiertes Wasser.

Die so erhaltene Giessmasse wird mit einem Rakelgussgerät in einer Dicke von 24 µm auf eine transparente Polyesterfolie aufgetragen und trocknen gelassen. Danach wird eine Schutzschicht aus Gelatine durch Eintauchen der Folie in Gelatine appliziert.

b) Bestimmung der Fluoreszenz

Die Messung der Fluoreszenz der so hergestellten Folienproben erfolgt in einem ®Shimadzu UV-240 UV-Vis.-Spektrophotometer, der mit einer Integrierkugel (Ulbricht'sche Sphäre) ausgestattet ist. Nach der automatischen Eichung mit Bariumsulfat als Reflektor in beiden Kanälen wird ein UV-Filter (Kodak®Wratten 2C) zwischen der Kugel und der Photozelle eingebaut; dann wird eine Basislinie-Korrektur zwischen 900 und 420 nm durchgeführt. Bei 470 nm wird die Absorption auf Null eingestellt; anschliessend können Messsungen im Energiemodus (Spaltbreite 5 nm) zwischen 420 und 300 nm durchgeführt werden. Zur Auswertung wird das erste Maximum unterhalb 380 nm herangezogen.

Diese Messeinrichtung erlaubt es, in einfacher Weise ein Mass für die Totalfluoreszenz als Funktion der Anregungsfrequenz zu erhalten. Unterhalb 380 nm wird das Anregungslicht vom UV-Filter total absorbiert, so dass die Messwerte dort genau sind. Zwischen 380 und 420 nm sind keine genauen Messungen möglich, da das Anregungslicht dort nur unvollständig gefiltert wird.

Alle Werte werden im Vergleich zu einer Standardprobe gemessen und sind in der nachfolgenden Tabelle 6 zusammengefasst.

Tabelle 6

| Verbindung gemäss Beispiel | 36 | 38 | 42 | 43 | 57 | 58 | *) |
|---|---|---|---|---|---|---|---|
| Fluoreszenz | 128 | 114 | 134 | 111 | 127 | 120 | 100 |

*) Standard

Beispiel 83:

Nach dem in Beispiel 82 angegebenen Verfahren werden Gelatineschichten hergestellt, die eine Reihe weiterer Verbindungen der Formel I enthalten. Danach wird die Fluoreszenzquantenausbeute der optischen Aufheller in der Gelatineschicht auf dem Polyesterträger nach der Methode von Eitle-Ganz (siehe Textilveredlung 3, 389-392 (1968)) bestimmt. Die erhaltenen Resultate sind in der nachfolgenden Tabelle 7 zusammengefasst.

Tabelle 7

| Verbindung gemäss Beispiel | Fluoreszenzquantenausbeute nach Eitle-Ganz (in %) |
|---|---|
| 36 | 23,4 |
| 38 | 27,0 |
| 41 | 20,7 |
| 42 | 23,3 |
| 43 | 22,2 |
| 48 | 22,4 |
| 56 | 25,3 |
| 57 | 23,1 |
| 58 | 23,8 |
| 64 | 25,2 |
| 71 | 26,1 |
| 72 | 21,9 |
| 75 | 22,6 |
| 80 | 23,4 |
| 81 | 25,1 |

Beispiel 84: Herstellung von Polyethylenfolien

Polyethylengranulat (LDPE, ®Coethylene HL 2578) wird jeweils mit 0,25 % (bezogen auf das Polyethylengewicht) einer Verbindung gemäss Beispiel 36 bzw. 37 trocken vermischt. Auf einem Schwabenthan-Extruder wird dann bei 140°C die Mischung zu einer ca. 30 µm dicken Folie verarbeitet. Die Fluoreszenz wird wie in Beispiel 82 beschrieben gemessen. Es zeigt sich, dass der Polyethylenfilm eine hohe Fluoreszenz aufweist.

Beispiel 85:

a) 1000 g Polyestergranulat vom Typ ®Terlenka matt (enthaltend 0,5 % $TiO_2$), GeO-Typ, wird im Trockenschrank ca. 16 Stunden bei 110°C und anschliessend 2 Stunden bei 160°C getrocknet, dann auf 70°C abgekühlt und in vorgewärmte Normschliffflaschen eingefüllt. Dann werden jeweils 5 g der zu prüfenden Verbindung der Formel I hinzugefügt, die Flaschen dicht verschlossen und nach vollständigem Abkühlen auf dem Röhnradmischer so lange gemischt, bis die Verbindung der Formel I gleichförmig über das Granulat verteilt ist (Kontrolle unter der UV-Lampe). Die Verbindungen der Formel I werden vor der Zugabe zum Granulat in einem Mörser fein pulverisiert.

Danach wird das so behandelte Polyestergranulat zu Fäden versponnen. Die Flaschen werden erst unmittelbar vor dem Verspinnen geöffnet. Das Verspinnen erfolgt in einer Fourné-Spinnanlage unter folgenden Bedingungen: Spinntemperatur 275°C/280°C/285°C; Abzug: 500 m/Min.; Düse: 34 Löcher/0,25 mm; Verstreckung: 1:4 bei 100°C; Endtiter: 130/34 den.; Avivage: ®Limazol ZE, 15 % in Wasser.

b) Die gemäss a) erhaltenen Fäden werden in 12facher Lage auf einen Karton 40 x 170 mm in einer Breite von 40 mm aufgewickelt. Die Fäden werden in einem Atlas Weatherometer Ci65 bei einer Schwarztafeltemperatur von 63 ± 3°C nach ASTM 626-77 bestrahlt. Nach den in der Tabelle 8 angegebenen Zeiträumen werden Proben entnommen und ihr Yellowness-Index (YI, Mass für die Verfärbung) mit einem MacBeth-Kolorimeter nach ASTM D 72-1920 bestimmt. Die Werte sind der nachfolgenden Tabelle 8 zu entnehmen.

Tabelle 8

| Verbindung gemäss Beispiel | YI nach Belichtungszeit in Stunden | | | | | | |
|---|---|---|---|---|---|---|---|
| | 0 | 7 | 25 | 125 | 205 | 345 | 463 |
| | Stunden | | | | | | |
| 36 | -1,4 | -2,9 | -3,2 | -2,8 | -2,1 | -1,0 | 0 |
| 53 | -1,3 | -2,6 | -2,9 | -2,2 | -1,3 | -0,3 | 0,7 |

c) Die gemäss a) erhaltenen Fäden werden in 12facher Lage auf einen Karton 40 x 170 mm in einer Breite

von 40 mm aufgewickelt. Der Weissgrad der Fäden wird in einem Spektralphotometer RFC-3 der Fa. Zeiss bestimmt. Es werden die Weissgradwerte nach dem CIBA-GEIGY-Weissmassstab (vgl. CIBA-GEIGY-Rundschau 1973/1, Seiten 10-12) berechnet. Die erhaltenen Werte sind der nachfolgenden Tabelle 9 zu entnehmen.

Tabelle 9

| Verbindung gemäss Beispiel | 36 | 53 |
|---|---|---|
| Weissgrad | 163 | 155 |

d) Die gemäss a) erhaltenen Fäden werden zu Wirkstrümpfen verarbeitet, die aus drei Filamenten zusammen hergestellt werden. Diese weden in 4-facher Lage auf einen Blechstreifen 40 x 170 mm aufgewickelt. Die Lichtechtheit der in den Fäden enthaltenen optischen Aufheller der Formel I wird gemäss Normvorschrift ISO 105/B02, 1984 (Belichtung mit Xenon-Lampe und Vergleich mit dem Blaumassstab) bestimmt. Die erhaltenen Werte sind in Tabelle 10 enthalten.

Tabelle 10

| Verbindung gemäss Beispiel | 36 | 53 |
|---|---|---|
| Lichtechtheit | >7 | >7 |

## Patentansprüche

1. Verbindungen der Formel

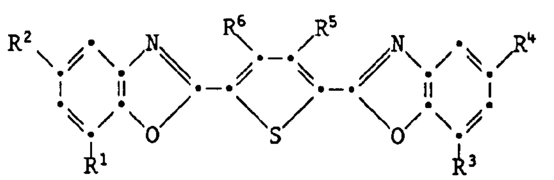

(I) ,

worin
$R^1$ und $R^3$ unabhängig voneinander $C_1$-$C_8$-Alkyl, Cyclopentyl, Cyclohexyl, $C_7$-$C_9$-Aralkyl, Phenyl oder eine Gruppe der Formel

$$-C_mH_{2m+1-q}(X)_q \qquad (II)$$

bedeuten,
worin m für eine Zahl von 0-10 und q für 1 oder 2 stehen, wobei im Fall m = 0, q = 1 ist, $R^2$ und $R^4$ unabhängig voneinander jeweils eine Gruppe der Formel II bedeuten,
oder $R^2$ und $R^4$ für den Fall, dass $R^1$ und $R^3$ jeweils eine Gruppe der Formel II darstellen, unabhängig voneinander auch $C_1$-$C_8$-Alkyl, Cyclopentyl, Cyclohexyl, $C_7$-$C_9$-Aralkyl oder Phenyl bedeuten können,
$R^5$ und $R^6$ unabhängig voneinander Wasserstoff, Methyl, Ethyl oder zusammen Trimethylen oder Tetramethylen bedeuten,
und X

a) eine Gruppe der Formel -$COOR^9$, worin $R^9$ Wasserstoff, ein Alkalimetallion oder das Aequivalent eines Erdalkalimetallions, $C_1$-$C_{30}$-Alkyl, $C_1$-$C_{18}$-Hydroxyalkyl, durch mindestens ein -O- unterbrochenes $C_3$-$C_{20}$-Alkyl oder -Hydroxyalkyl, unsubstituiertes oder durch OH oder/und $C_1$-$C_4$-Alkyl substituiertes $C_5$-$C_{12}$-Cycloalkyl, $C_2$-$C_{30}$-Alkenyl, $C_7$-$C_{15}$-Aralkyl, Glycidyl, Furfuryl, oder eine Gruppe -$CH_2$-CH(OH)-$R^{10}$ darstellt, worin $R^{10}$ für Wasserstoff, $C_1$-$C_{12}$-Alkyl, $C_7$-$C_9$-Aralkyl oder -$CH_2OR^{11}$ steht, wobei $R^{11}$ $C_1$-

$C_{18}$-Alkyl, Cyclohexyl, Phenyl, Tolyl oder Benzyl ist, oder $R^9$ eine Gruppe der Formel -$(CH_2)_pP(O)(OR^{16})(OR^{17})$ oder -$(CH_2)_pP(O)(OR^{16})$-$R^{18}$, worin p 1 bis 6 ist und $R^{16}$, $R^{17}$ und $R^{18}$ wie nachstehend unter d) definiert sind, darstellt;

b) eine Gruppe der Formel -$CONR^{12}R^{13}$, worin $R^{12}$ und $R^{13}$ unabhängig voneinander Wasserstoff, $C_1$-$C_{18}$-Alkyl, durch mindestens ein -O- unterbrochenes $C_3$-$C_{20}$-Alkyl, unsubstituiertes oder durch $C_1$-$C_4$-Alkyl oder/und OH substituiertes $C_5$-$C_{12}$-Cycloalkyl, Phenyl oder durch $C_1$-$C_{12}$-Alkyl, $C_1$-$C_4$-Alkoxy oder/und Halogen substituiertes Phenyl, $C_3$-$C_{18}$-Alkenyl, $C_7$-$C_{12}$-Aralkyl oder $C_2$-$C_4$-Hydroxyalkyl sind, oder $R^{12}$ und $R^{13}$ zusammen $C_4$-$C_5$-Alkylen oder durch -O- oder -$N(R^{15})$ unterbrochenes $C_4$-$C_6$-Alkylen darstellen, wobei $R^{15}$ Wasserstoff, $C_1$-$C_{18}$-Alkyl, Cyclohexyl, $C_2$-$C_6$-Alkenyl, Phenyl oder $C_7$-$C_{12}$-Alkylphenyl oder $C_7$-$C_{12}$-Aralkyl bedeutet;

c) eine Gruppe der Formel

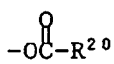

,

worin $R^{20}$ $C_1$-$C_{18}$-Alkyl, Cyclohexyl, $C_2$-$C_{18}$-Alkenyl, Phenyl oder $C_7$-$C_{12}$-Alkylphenyl oder $C_7$-$C_{12}$-Aralkyl bedeutet;

d) eine Gruppe der Formel -$P(O)(OR^{16})(OR^{17})$ oder -$P(O)(OR^{16})$-$R^{18}$, worin $R^{16}$ Wasserstoff oder $C_1$-$C_{12}$-Alkyl, $R^{17}$ $C_1$-$C_{12}$-Alkyl und $R^{18}$ $C_1$-$C_{12}$-Alkyl oder Phenyl darstellen; oder

e) eine Gruppe der Formel

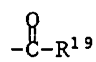

,

worin $R^{19}$ $C_1$-$C_{12}$-Alkyl darstellt, bedeutet.

2. Verbindungen nach Anspruch 1, worin $R^1$ und $R^3$ sowie $R^2$ und $R^4$ jeweils gleich sind.

3. Verbindungen nach Anspruch 1 oder 2, worin $R^1$ bzw. $R^3$ $C_1$-$C_4$-Alkyl, Cyclohexyl oder eine Gruppe der Formel II bedeutet.

4. Verbindungen nach Anspruch 1, worin $R^1$ und $R^3$ $C_1$-$C_4$-Alkyl, Cyclohexyl, Benzyl oder eine Gruppe der Formel II, $R^2$ und $R^4$ eine Gruppe der Formel II, oder, wenn $R^1$ für eine Gruppe der Formel II steht, auch $C_1$-$C_8$-Alkyl, Cyclopentyl, Cyclohexyl oder Phenyl-$C_1$-$C_3$-alkyl, m 0 bis 6, $R^5$ und $R^6$ unabhängig voneinander Wasserstoff, Methyl oder Ethyl, $R^9$ Wasserstoff, $C_1$-$C_{18}$-Alkyl, Cyclopentyl, Cyclohexyl, $C_2$-$C_{18}$-Alkenyl, Phenyl-$C_1$-$C_3$-alkyl, Furfuryl, durch ein bis 4 -O- unterbrochenes $C_3$-$C_{18}$-Alkyl oder -$(CH_2)_pP(O)(OC_1$-$C_6$-$Alkyl)_2$, worin p 1 bis 6 ist, $R^{12}$ und $R^{13}$ unabhängig voneinander Wasserstoff, $C_1$-$C_{18}$-Alkyl, Cyclopentyl, Cyclohexyl, Phenyl, durch $C_1$-$C_4$-Alkyl, Methoxy oder/und Chlor substituiertes Phenyl, $C_3$-$C_{18}$-Alkenyl, Benzyl oder $C_2$-$C_4$-Hydroxyalkyl, oder $R^{12}$ und $R^{13}$ zusammen $C_4$-$C_5$-Alkylen oder durch -O-, -NH- oder -$N(CH_3)$- unterbrochenes $C_4$-$C_5$-Alkylen, $R^{15}$ $C_1$-$C_8$-Alkyl, Cyclohexyl, $C_2$-$C_6$-Alkenyl, Phenyl, Tolyl oder Benzyl, $R^{16}$ Wasserstoff oder $C_1$-$C_8$-Alkyl, $R^{17}$ $C_1$-$C_8$-Alkyl, $R^{18}$ $C_1$-$C_8$-Alkyl und $R^{19}$ $C_1$-$C_8$-Alkyl bedeuten.

5. Verbindungen nach einem der Ansprüche 1-4, worin X eine Gruppe der Formel -$COOR^9$ oder -$CONR^{12}R^{13}$ ist.

6. Verbindungen nach einem der Ansprüche 1-5, worin $R^2$ bzw. $R^4$ eine Gruppe der Formel II ist, worin q für 1 oder 2 und m für 0 bis 6 stehen, oder $R^2$ bzw. $R^4$ $C_1$-$C_8$-Alkyl, Cyclopentyl, Cyclohexyl oder Phenyl-$C_1$-$C_3$-alkyl bedeutet, wenn $R^1$ bzw. $R^3$ eine Gruppe der Formel II ist.

7. Verbindungen nach Anspruch 1, worin $R^1$ und $R^3$ gleich sind und $C_1$-$C_4$-Alkyl oder eine Gruppe der Formel II bedeuten, $R^2$ und $R^4$ gleich sind und für eine Gruppe der Formel II oder, wenn $R^1$ und $R^3$ Gruppen der Formel II sind, auch für $C_1$-$C_4$-Alkyl stehen, m 0-6 und X eine Gruppe der Formel -$COOR^9$ oder -$CONR^{12}R^{13}$ bedeuten, worin $R^9$ Wasserstoff, $C_1$-$C_{18}$-Alkyl, $C_3$-$C_{18}$-Alkenyl, Furfuryl, Cyclohexyl, Phenyl-$C_1$-$C_3$-alkyl, durch ein bis 4 -O- unterbrochenes $C_3$-$C_{18}$-Alkyl oder -$(CH_2)_{p'}P(O)(OC_1$-$C_4$-$Alkyl)_2$ ist, worin

$_{p'}$ 1 bis 4 bedeutet und $R^{12}$ und $R^{13}$ unabhängig voneinander Wasserstoff oder $C_1$-$C_{12}$-Alkyl sind.

8. Organisches Material, enthaltend als optischen Aufheller mindestens eine Verbindung der Formel I gemäss Anspruch 1.

9. Material nach Anspruch 8, dadurch gekennzeichnet, dass das Material ein synthetisches Polymer ist.

10. Material nach Anspruch 9, dadurch gekennzeichnet, dass es ein Ueberzugsmaterial, vorzugsweise eine Lackformulierung, ist.

11. Material nach Anspruch 8, dadurch gekennzeichnet, dass es eine Druckfarbe oder Drucktinte ist.

12. Material nach Anspruch 8, dadurch gekennzeichnet, dass das Material ein Abbildungs- oder Aufzeichnungsmaterial, insbesondere ein photographisches Aufzeichnungsmaterial, vorzugsweise ein Photopapier ist.

13. Material nach einem der Ansprüche 8-11, dadurch gekennzeichnet, dass es die Verbindung(en) der Formel (I) in einer Menge von 0,0001 bis 2 Gew.-% enthält.

14. Material nach Anspruch 12, dadurch gekennzeichnet, dass das Material eine Bildaufnahmeschicht bzw. ein Aufzeichnungsmaterial für den Tintenstrahl-, Farbstoffdiffusionstransfer-, thermischen Wachstransfer-, und Punktmatrix-Druck oder für Verwendung mit elektrostatischen, elektrographischen, elektrophoretischen, magnetographischen und laserelektrophotographischen Druckern ist.

15. Material nach Anspruch 12, dadurch gekennzeichnet, dass das Material ein druckempfindliches Papier oder ein Aufzeichnungsmaterial ist, das auf den Prinzipien der Photopolymerisation, Photoplastifizierung, hitze- oder lichtempfindlichen Diazoniumsalze, Leukofarbstoffe und Oxidationsmittel, Farbstofflactone plus Lewis-Säure, oder des Aufbrechens von Farbstoff oder farblose Farbbildner enthaltender Mikrokapseln beruht.

16. Material nach Anspruch 12, dadurch gekennzeichnet, dass es eine Verstärkerfolie oder Strahlungsspeicherfolie für die medizinische Radiologie ist, die mit Licht anregbar ist.

17. Material nach einem der Ansprüche 12 oder 14 bis 16, dadurch gekennzeichnet, dass es die Verbindung(en) der Formel I in einer Menge von 1-500, insbesondere 2-250 mg pro Quadratmeter des jeweiligen Materials enthält.

18. Verfahren zum optischen Aufhellen von organischen Materialien, dadurch gekennzeichnet, dass man mindestens eine Verbindung der Formel I gemäss Anspruch 1 diesen Materialien einverleibt oder auf diese aufbringt.

19. Verwendung von Verbindungen der Formel I gemäss Anspruch 1 als optische Aufheller.

20. Verwendung nach Anspruch 19 als optische Aufheller für synthetische Polymere und Abbildungs- und Aufzeichnungsmaterialien.

## Claims

1. A compound of the formula

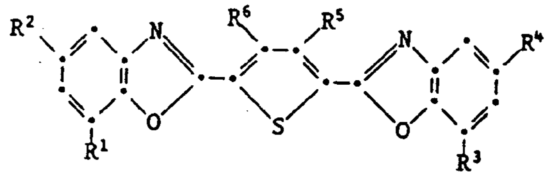

(I)

in which $R^1$ and $R^3$ independently of one another are $C_1$-$C_8$alkyl, cyclopentyl, cyclohexyl, $C_7$-$C_9$aralkyl, phenyl or a group of the formula

$$-C_mH_{2m+1-q}(X)_q \qquad (II)$$

in which m is a number from 0-10 and q is 1 or 2, with q = 1 in the case of m = 0, $R^2$ and $R^4$ independently of one another are each a group of the formula II or, in the case that $R^1$ and $R^3$ are each a group of the formula II, $R^2$ and $R^4$ independently of one another can also be $C_1$-$C_8$alkyl, cyclopentyl, cyclohexyl, $C_7$-$C_9$aralkyl or phenyl, $R^5$ and $R^6$ independently of one another are hydrogen, methyl, ethyl or together trimethylene or tetramethylene, and X is

a) a group of the formula $-COOR^9$, in which $R^9$ is hydrogen, an alkali metal ion or the equivalent of an alkaline earth metal ion, $C_1$-$C_{30}$alkyl, $C_1$-$C_{18}$hydroxyalkyl, $C_3$-$C_{20}$alkyl or $C_3$-$C_{20}$hydroxyalkyl interrupted by at least one -O-, $C_5$-$C_{12}$cycloalkyl which is unsubstituted or substituted by OH or/and $C_1$-$C_4$alkyl, $C_2$-$C_{30}$alkenyl, $C_7$-$C_{15}$aralkyl, glycidyl, furfuryl or a group $-CH_2-CH(OH)-R^{10}$, in which $R^{10}$ is hydrogen, $C_1$-$C_{12}$alkyl, $C_7$-$C_9$aralkyl or $-CH_2OR^{11}$, $R^{11}$ being $C_1$-$C_{18}$alkyl, cyclohexyl, phenyl, tolyl or benzyl, or $R^9$ is a group of the formula $-(CH_2)_pP(O)(OR^{16})(OR^{17})$ or $-(CH_2)_pP(O)(OR^{16})-R^{18}$, in which p is 1 to 6 and $R^{16}$, $R^{17}$ and $R^{18}$ are as defined below under d),

b) a group of the formula $-CONR^{12}R^{13}$, in which $R^{12}$ and $R^{13}$ independently of one another are hydrogen, $C_1$-$C_{18}$alkyl, $C_3$-$C_{20}$alkyl interrupted by at least one -O-, $C_5$-$C_{12}$cycloalkyl which is unsubstituted or substituted by $C_1$-$C_4$alkyl or/and OH, phenyl or phenyl substituted by $C_1$-$C_{12}$alkyl, $C_1$-$C_4$alkoxy or/and halogen, $C_3$-$C_{18}$alkenyl, $C_7$-$C_{12}$aralkyl or $C_2$-$C_4$hydroxyalkyl, or $R^{12}$ and $R^{13}$ together are $C_4$-$C_5$alkylene or $C_4$-$C_6$alkylene interrupted by -O- or $-N(R^{15})$, $R^{15}$ being hydrogen, $C_1$-$C_{18}$alkyl, cyclohexyl, $C_2$-$C_6$alkenyl, phenyl or $C_7$-$C_{12}$alkylphenyl or $C_7$-$C_{12}$aralkyl,

c) a group of the formula

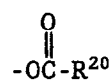,

in which $R^{20}$ is $C_1$-$C_{18}$alkyl, cyclohexyl, $C_2$-$C_{18}$alkenyl, phenyl or $C_7$-$C_{12}$alkylphenyl or $C_7$-$C_{12}$aralkyl,

d) a group of the formula $-P(O)(OR^{16})(OR^{17})$ or $-P(O)(OR^{16})-R^{18}$, where $R^{16}$ is hydrogen or $C_1$-$C_{12}$alkyl, $R^{17}$ is $C_1$-$C_{12}$alkyl and $R^{18}$ is $C_1$-$C_{12}$alkyl or phenyl, or

e) a group of the formula

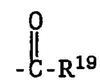,

in which $R^{19}$ is $C_1$-$C_{12}$alkyl.

2. A compound according to claim 1, wherein $R^1$ and $R^3$, and $R^2$ and $R^4$ respectively are identical.

3. A compound according to either of claims 1 or 2, wherein $R^1$ and/or $R^3$ are $C_1$-$C_4$alkyl, cyclohexyl or a group of the formula II.

4. A compound according to claim 1, in which $R^1$ and $R^3$ are $C_1$-$C_4$alkyl, cyclohexyl, benzyl or a group of the formula II, $R^2$ and $R^4$ are a group of the formula II or, if $R^1$ is a group of the formula II, are also $C_1$-$C_8$alkyl, cyclopentyl, cyclohexyl or phenyl-$C_1$-$C_3$alkyl, m is 0 to 6, $R^5$ and $R^6$ independently of one another are hydrogen, methyl or ethyl, $R^9$ is hydrogen, $C_1$-$C_{18}$alkyl, cyclopentyl, cyclohexyl, $C_2$-$C_{18}$alkenyl, phenyl-$C_1$-$C_3$alkyl, furfuryl, $C_3$-$C_{18}$alkyl interrupted by 1 to 4 -O- or $-(CH_2)_pP(O)(OC_1$-$C_6alkyl)_2$ in which p is 1 to 6, $R^{12}$ and $R^{13}$ independently of one another are hydrogen, $C_1$-$C_{18}$alkyl, cyclopentyl, cyclohexyl, phenyl, phenyl substituted by $C_1$-$C_4$alkyl, methoxy or/and chlorine, $C_3$-$C_{18}$alkenyl, benzyl or $C_2$-$C_4$hydroxyalkyl, or $R^{12}$ and $R^{13}$ together are $C_4$-$C_5$alkylene or $C_4$-$C_5$alkylene interrupted by -O-, -NH- or $-N(CH_3)-$, $R^{15}$ is $C_1$-$C_8$alkyl, cyclohexyl, $C_2$-$C_6$alkenyl, phenyl, tolyl or benzyl, $R^{16}$ is hydrogen or $C_1$-$C_8$alkyl, $R^{17}$ is $C_1$-$C_8$alkyl, $R^{18}$ is $C_1$-$C_8$alkyl and $R^{19}$ is $C_1$-$C_8$alkyl.

5. A compound according to any one of claims 1-4, wherein X is a group of the formula $-COOR^9$ or $-CONR^{12}R^{13}$.

6. A compound according to any one of claims 1-5, wherein $R^2$ and/or $R^4$ are a group of the formula II, in which q is 1 or 2 and m is 0 to 6, or, if $R^1$ and/or $R^3$ are a group of the formula II, $R^2$ and/or $R^4$ are $C_1$-

$C_8$alkyl, cyclopentyl, cyclohexyl or phenyl-$C_1$-$C_3$alkyl.

7. A compound according to claim 1, wherein $R^1$ and $R^3$ are identical and are $C_1$-$C_4$alkyl or a group of the formula II, $R^2$ and $R^4$ are identical and are a group of the formula II, or, if $R^1$ and $R^3$ are groups of the formula II, can also be $C_1$-$C_4$alkyl, m is 0-6 and X is a group of the formula -$COOR^9$ or -$CONR^{12}R^{13}$, in which $R^9$ is hydrogen, $C_1$-$C_{18}$alkyl, $C_3$-$C_{18}$alkenyl, furfuryl, cyclohexyl, phenyl-$C_1$-$C_3$alkyl, $C_3$-$C_{18}$alkyl interrupted by one to 4 -O- or -$(CH_2)_{p'}P(O)(OC_1$-$C_4alkyl)_2$ in which p' is 1 to 4, and $R^{12}$ and $R^{13}$ independently of one another are hydrogen or $C_1$-$C_{12}$alkyl.

8. An organic material, which contains, as a fluorescent brightener, at least one compound of the formula I according to claim 1.

9. A material according to claim 8, which is a synthetic polymer.

10. A material according to claim 9, which is a coating material, preferably a lacquer formulation.

11. A material according to claim 8, which is a printing colour or printing ink.

12. A material according to claim 8, which is an imaging or recording material, especially a photographic recording material, preferably a photographic paper.

13. A material according to any one of claims 8-11, which contains the compound(s) of the formula (I) in a quantity of 0.0001 to 2 % by weight.

14. A material according to claim 12, which is an image-receiving layer or a recording material for ink-jet printing, dye diffusion transfer printing, thermal wax transfer printing and dot matrix printing or for use with electrostatic, electrographic, electrophoretic, magnetographic and laser-electrophotographic printers.

15. A material according to claim 12, which is a pressure-sensitive paper or a recording material which is based on the principles of photopolymerization, photoplasticization, heat- or light-sensitive diazonium salts, leuco dyes and oxidizing agents, dye lactones plus Lewis acid, or on the principle of breaking up microcapsules containing a dye or colourless colour formers.

16. A material according to claim 12, which is an intensifying film or radiation storage film for medical radiology and can be excited by light.

17. A material according to any one of claims 12 or 14 to 16, which contains the compound(s) of the formula I in a quantity of 1-500 and especially 2-250 mg per square metre of the particular material.

18. A method for fluorescent brightening of organic materials, which comprises incorporating at least one compound of the formula I according to claim 1 into these materials or applying them thereto.

19. The use of a compound of the formula I according to claim 1 as a fluorescent brightener.

20. The use according to claim 19 as a fluorescent brightener for synthetic polymers and imaging and recording materials.

## Revendications

1. Composés de formule

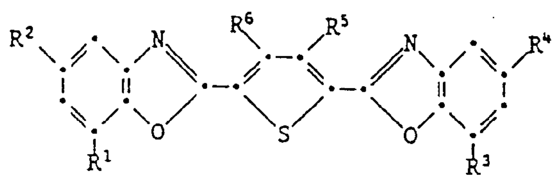

(I) ,

dans laquelle

$R^1$ et $R^3$ représentent chacun, indépendamment l'un de l'autre, un alkyle en $C_1$-$C_8$, le groupe cyclopentyle ou cyclohexyle, un aralkyle en $C_7$-$C_9$, le groupe phényle ou un groupe de formule

$$-C_mH_{2m+1-q}(X)_q \qquad (II)$$

m étant un nombre de 0 à 10 et q le nombre 1 ou 2, avec la condition que si m = 0, q = 1,
$R^2$ et $R^4$ sont chacun, indépendamment l'un de l'autre, un groupe de formule II ci-dessus,
ou bien $R^2$ et $R^4$, si $R^1$ et $R^3$ sont chacun un groupe de formule II, peuvent également représenter chacun, indépendamment l'un de l'autre, un alkyle en $C^1$-$C^8$, le groupe cyclopentyle ou cyclohexyle, un aralkyle en $C^7$-$C^9$ ou le groupe phényle,
$R^5$ et $R^6$ représentent chacun, indépendamment l'un de l'autre, l'hydrogène ou le groupe méthyle ou éthyle ou bien forment ensemble un groupe triméthylène ou tétraméthylène, et X désigne :

a) un groupe $-COOR^9$, $R^9$ étant l'hydrogène ou bien un ion de métal alcalin ou l'équivalent d'un ion de métal alcalino-terreux, un alkyle en $C_1$-$C_{30}$, un hydroxyalkyle en $C_1$-$C_{18}$, un alkyle ou un hydroxyalkyle en $C_3$-$C_{20}$ interrompus par au moins un atome d'oxygène, sans substituants ou avec un groupe OH et/ou un alkyle en $C_1$-$C_4$, un cycloalkyle en $C_5$-$C_{12}$, un alcényle en $C_2$-$C_{30}$, un aralkyle en $C_7$-$C_{15}$, un groupe glycidyle ou furfuryle ou encore un groupe $-CH_2-CH(OH)-R^{10}$, $R^{10}$ désignant l'hydrogène, un alkyle en $C_1$-$C_{12}$, un aralkyle en $C_7$-$C_9$ ou un groupe $-CH_2OR^{11}$, $R^{11}$ étant un alkyle en $C_1$-$C_{18}$, ou un groupe cyclohexyle, phényle, tolyle ou benzyle, ou bien $R^9$ est un groupe $-(CH2)_pP(O)(OR^{16})(OR^{17})$ ou $-(CH2)_pP(O)(OR^{16})-R^{18}$, dans lesquels p est un nombre de 1 à 6 et $R^{16}$, $R^{17}$ et $R^{18}$ ont les significations indiquées ci-dessous en d) ; ou bien
b) un groupe $-CONR^{12}R^{13}$,
$R^{12}$ et $R^{13}$ désignant chacun, indépendamment l'un de l'autre, un alkyle en $C_1$-$C_{18}$, un alkyle en $C_3$-$C_{20}$ interrompu par un ou plusieurs atomes d'oxygène, sans substituants ou avec un alkyle en $C_1$-$C_4$ et/ou un groupe OH, un cycloalkyle en $C_5$-$C_{12}$, un phényle pouvant être éventuellement substitué par un alkyle en $C_1$-$C_{12}$, un alcoxy en $C_1$-$C_4$ et/ou par un halogène, un alcényle en $C_3$-$C_{18}$, un aralkyle en $C_7$-$C_{12}$ ou encore un hydroxyalkyle en $C_2$-$C_4$, ou bien $R^{12}$ et $R^{13}$ forment ensemble un alkylène en $C_4$ ou $C_5$ ou un alkylène en $C_4$-$C_6$ interrompu par un atome d'oxygène ou par un groupe $-N(R^{15})$, $R^{15}$ désignant l'hydrogène, un alkyle en $C_1$-$C_{18}$, un cyclohexyle, un alcényle en $C_2$-$C_6$, un phényle ou un alkylphényle à alkyle en $C_7$-$C_{12}$, ou encore un aralkyle en $C_7$-$C_{12}$ ;
c) un groupe

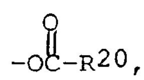

$R^{20}$ désignant un alkyle en $C_1$-$C_{18}$, un cyclohexyle, un alcényle en $C_2$-$C_{18}$, un phényle ou un alkylphényle à alkyle en $C_7$-$C_{12}$ ou un aralkyle en $C_7$-$C_{12}$ ;
d) un groupe $-P(O)(OR^{16})(OR^{17})$ ou $-P(O)(OR^{16})-R^{18}$ dans lesquels $R^{16}$ est l'hydrogène ou un alkyle en $C_1$-$C_{12}$, $R^{17}$ un alkyle en $C_1$-$C_{12}$ et $R^{18}$ un alkyle en $C_1$-$C_{12}$ ou un phényle ; ou encore
e) un groupe

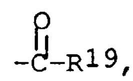

$R^{19}$ étant un alkyle en $C_1$-$C_{12}$.

2. Composés selon la revendication 1 dans lesquels $R^1$ et $R^3$ sont identiques, ainsi que $R^2$ et $R^4$.

3. Composés selon la revendication 1 ou 2 dans lesquels $R^1$ et/ou $R^3$ sont des alkyles en $C^1$-$C^4$, le groupe cyclohexyle ou un groupe de formule II.

4. Composés selon la revendication 1 dans lesquels $R^1$ et $R^3$ sont un alkyle en $C_1$-$C_4$, un cyclohexyle, un benzyle ou un groupe de formule II, $R^2$ et $R^4$ sont un groupe de formule II ou bien, si $R^1$ est un groupe de formule II, peuvent être aussi un alkyle en $C_1$-$C_8$, le groupe cyclopentyle ou cyclohexyle ou un phényl-$C_1$-$C_3$-alkyle, m est un nombre de 0 à 6, $R^5$ et $R^6$ désignent chacun, indépendamment l'un de l'autre, l'hydrogène ou le groupe méthyle ou éthyle, $R^9$ représente l'hydrogène, un alkyle en $C_1$-$C_{18}$, le groupe cyclopentyle ou cyclohexyle, un alcényle en $C_2$-$C_{18}$, un phényl-$C_1$-$C_3$-alkyle, le groupe furfuryle, un alkyle en $C_3$-$C_{18}$ interrompu par 1 à 4 atomes d'oxygène ou un groupe $-(CH_2)_pP(O)(OC_1\text{-}C_6\text{-alkyle})_2$, p étant un

nombre de 1 à 6, $R^{12}$ et $R^{13}$ représentent chacun, indépendamment l'un de l'autre, l'hydrogène, un alkyle en $C_1$-$C_{18}$, un groupe cyclopentyle, cyclohexyle ou phényle, ou un phényle substitué par un alkyle en $C_1$-$C_4$, le groupe méthoxy et/ou un atome de chlore, un alcényle en $C_3$-$C_{18}$, un benzyle ou un hydroxyalkyle en $C_2$-$C_4$, ou bien $R^{12}$ et $R^{13}$ forment ensemble un alkylène en $C_4$ ou $C_5$ pouvant être éventuellement interrompu par un atome d'oxygène ou un groupe -NH- ou -N($CH_3$)-, $R^{15}$ désigne un alkyle en $C_1$-$C_8$, un cyclohexyle, un alcényle en $C_2$-$C_6$, un phényle, tolyle ou benzyle, $R^{16}$ l'hydrogène ou un alkyle en $C_1$-$C_8$, et $R^{17}$, $R^{18}$ et $R^{19}$ sont chacun un alkyle en $C_1$-$C_8$.

**5.** Composés selon l'une des revendications 1 à 4 dans lesquels X est un groupe -$COOR^9$ ou -$CONR^{12}R^{13}$.

**6.** Composés selon l'une des revendications 1 à 5 dans lesquels $R^2$ et/ou $R^4$ sont un groupe de formule II, q étant le nombre 1 ou 2 et m un nombre de 0 à 6, ou bien sont un alkyle en $C_1$-$C_8$, le groupe cyclopentyle ou cyclohexyle ou un phényl-$C_1$-$C_3$-alkyle, si $R^1$ et/ou $R^3$ sont un groupe de formule II.

**7.** Composés selon la revendication 1 dans lesquels $R^1$ et $R^3$ sont identiques et sont chacun un alkyle en $C^1$-$C^4$ ou un groupe de formule II, $R^2$ et $R^4$ sont également identiques et chacun un groupe de formule II ou bien, si $R^1$ et $R^3$ sont des groupes de formule II, peuvent être également des alkyles en $C_1$-$C_4$, m est un nombre de 0 à 6 et X un groupe -$COOR^9$ ou -$CONR^{12}R^{13}$, $R^9$ désignant l'hydrogène, un alkyle en $C_1$-$C_{18}$, un alcényle en $C_3$-$C_{18}$, le groupe furfuryle ou cyclohexyle, un phényl-$C_1$-$C_3$-alkyle, un alkyle en $C_3$-$C_{18}$ interrompu par un 1 à 4 atomes d'oxygène ou encore un groupe -$(CH_2)_{p'}P(O)(OC_1$-$C_4$-alkyle$)_2$, p' étant un nombre de 1 à 4, et $R^{12}$ et $R^{13}$ désignant chacun, indépendamment l'un de l'autre, l'hydrogène ou un alkyle en $C_1$-$C_{12}$.

**8.** Matières organiques contenant comme azureurs optiques un ou plusieurs composés de formule I selon les revendications précédentes.

**9.** Matière selon la revendication 8, caractérisée en ce qu'il s'agit d'une matière polymère synthétique.

**10.** Matière selon la revendication 9, caractérisée en ce qu'il s'agit d'un produit de revêtement, de préférence d'une formule de vernissage.

**11.** Matière selon la revendication 8, caractérisée en ce qu'il s'agit d'une couleur d'impression ou d'une encre d'impression.

**12.** Matière selon la revendication 8, caractérisée en ce qu'il s'agit d'une matière de formation d'images ou d'enregistrement, en particulier d'un produit d'enregistrement photographique et de préférence d'un papier photographique.

**13.** Matière selon l'une des revendications 8 à 11, caractérisée en ce qu'elle contient le ou les composés de formule I dans une proportion de 0,0001 à 2 % en poids.

**14.** Matière selon la revendication 12, caractérisée en ce qu'il s'agit d'une couche de prise d'images ou d'un produit d'enregistrement pour des impressions par encres, rayonnements, transfert de colorants par diffusion, transfert de cires à chaud ou par matrices à points, ou pour l'emploi avec des imprimantes électrostatiques, électrographiques, électrophorétiques, magnétographiques ou électrophotographiques à lasers.

**15.** Matière selon la revendication 12, caractérisée en ce qu'il s'agit d'un papier piézosensible ou d'un produit d'enregistrement reposant sur les principes de la photopolymérisation, de la photoplastification, de sels de diazonium thermosensibles ou photosensibles, de leucocolorants et agents d'oxydation, de colorants de lactones avec acides de Lewis, ou encore de l'ouverture ou rupture de microcapsules contenant un colorant ou un produit chromogène incolore.

**16.** Matière selon la revendication 12, caractérisée en ce qu'il s'agit d'une feuille de renforcement ou d'une feuille de stockage de rayonnement activable à la lumière pour la radiologie médicale.

**17.** Matière selon l'une des revendications 12 et 14 à 16, caractérisée en ce qu'elle contient le ou les composés de formule I dans une proportion par mètre carré de 1 à 500 mg, en particulier de 2 à 250 mg.

**18.** Procédé d'azurage optique de matières organiques, procédé caractérisé en ce que l'on incorpore à ces

matières ou on leur applique un ou plusieurs composés de formule I selon la revendication 1.

**19.** L'emploi de composés de formule I selon la revendication 1 comme azureurs optiques.

**20.** Emploi selon la revendication 19 comme azureurs optiques pour des polymères synthétiques ou des produits de formation et enregistrement d'images.